# EUROPEAN PATENT APPLICATION

(11) **EP 1 717 313 A1**
(43) Date of publication of application: **02.11.2006**
(21) Application number: 04787923.4
(22) Date of filing: 21.09.2004
(51) Int. Cl.: C12N 15/11, C12Q 1/68, G01N 33/53, G01N 33/566, C12M 1/00

(54) **NUCLEIC ACID PROBE, NUCLEIC ACID CHIP, METHOD OF DETECTING TARGET NUCLEIC ACID, METHOD OF SCREENING DRUG, APPARATUS FOR DETECTING TARGET NUCLEIC ACID AND GENE DIAGNOSIS METHOD**

(30) Priority: 22.09.2003 JP 2003329619
(71) Applicant: Kyoto University, Kyoto-shi, Kyoto 606-8501 (JP)
(72) Inventor: AOYAMA, Yasuhiro, c/o Kyoto University, Kyoto-shi, Kyoto 6158246 (JP); SANDO, Shinsuke, c/o Kyoto University, Kyoto-shi, Kyoto 6158246 (JP); SASAKI, Toshinori, c/o Kyoto University, Kyoto-shi, Kyoto 615824 (JP); NARITA, Atsushi, c/o Kyoto University, Kyoto-shi, Kyoto 6158246 (JP)
(74) Representative: Daniels, Jeffrey Nicholas
(86) International application number: PCT/JP2004/013740
(87) International publication number: WO 2005/028647

(57) **Abstract**

The nucleic acid probe of the invention is a nucleic acid probe for detecting a trace amount of target nucleic acid highly sensitively, highly accurately, and highly quickly, and is designed so that the nucleic acid probe undergoes a conformational change after hybridization to the target nucleic acid, the nucleic acid probe, which underwent the conformational change, has a decreased binding strength of hybridization and dissociates from the target nucleic acid. Further, the nucleic acid probe can hybridize to the target nucleic acid, and includes a cleavage portion, and a signal-generating portion from which a signal is generated, where the cleavage portion is cleaved upon hybridization to the target nucleic acid and generates a first probe fragment and a second probe fragment, at least one of which is capable of dissociating from the target nucleic acid. Further, the nucleic acid probe includes a cleavage portion, which is cleaved upon hybridization to the target nucleic acid, and has formed a locked structure as a result of intramolecular hybridization of the portions of the nucleic acid probe to each other until the nucleic acid probe hybridizes to the target nucleic acid.

## Description

### Technical Field

The present invention relates to nucleic acid probes, which have extremely high selectivity for target nucleic acids and can detect a trace amount of target nucleic acid highly sensitively and highly accurately by allowing amplification of the signal generated from nucleic acid probes hybridized to target nucleic acids, nucleic acid chips to which the nucleic acid probes were fixed; and methods for detecting target nucleic acids, methods for screening drugs, apparatuses for detecting target nucleic acids and gene diagnosis methods, in which the nucleic acid probes are used.

In recent years, techniques related to genomic information have been increasingly developed and particularly in the field of medicine, treatment of disease at molecular level has been becoming possible by the analysis of disease-related gene. In addition, tailor-made medicine corresponding to an individual patient has been becoming possible by gene diagnosis. For example, in the fields of pharmaceuticals, proteins such as antibodies or hormones are identified based on the genomic information, and are utilized as drugs. Furthermore, in the field of farming or the food-related fields, many products utilizing genomic information are created. In addition, the concept of gene diagnosis has been developed in which diagnosis is carried out by comparing the genomic information of a patient obtained using a nucleic acid probe, DNA chip, etc., with database by using a computer. Based on this gene diagnosis, such businesses as evaluation of the prognosis of disease, evaluation of risk of disease due to congenital factor, administration of tailor-made medicine, which could not be imagined before, are being performed.

Analysis of a trace amount of target nucleic acid by the nucleic acid probe or DNA chip used in the gene diagnosis is difficult. Thus, in order to carry out the gene diagnosis, the target nucleic acid must be amplified by PCR method. However, when the PCR method is used for amplification of a trace amount of the target nucleic acid, skills are required. Thus, previously, in many cases where PCR method is used, problem occurred that the target nucleic acid could not be detected due to inadequate amplification of the target nucleic acid. Development of a technique which overcomes this problem and can analyze a trace amount of target nucleic acid highly sensitively and highly quickly is essential for the development of the gene diagnosis. Such technique that can analyze a trace amount of target nucleic acid highly sensitively and highly quickly is required not only in the gene diagnosis but also in blood transfusion. When carrying out blood transfusion, a test whether or not blood is infected with viruses such as HIV, HBV, and HCV. In the case of immunological test such as a method using antibody beads or western blotting method, which are conventional methods adopted, there is a problem that generation of so-called window period cannot be prevented. Namely, in the immunological tests mentioned above, antibodies to the viruses are detected; however, the antibodies are not produced during one to two months after infection to the viruses, thus resulting in false negative of the immunological test in early phase of virus infection. Therefore, a technique is required that can analyze viruses (target nucleic acids), present only in trace amounts, e.g. in early phase of virus infection, highly sensitively and highly quickly in order to prevent the generation of the window period in the blood transfusion. Moreover, such technique is useful for early detection and/or early treatment of all kinds of infectious diseases, mental diseases, cancers, etc. and is extremely important.

Under such situation, a nucleic acid probe is disclosed which can detect a trace amount of target nucleic acid not by the PCR method, but by amplification of signal (See, Japanese Patent Application Laid-Open No. 2003-525631). In the case of this nucleic acid probe, a sensor molecule which hybridizes with a target nucleic acid and a reporter molecule, which is enzymatically cleaved due to DNAzyme activity of the sensor molecule and of which label is capable of generating a signal, are used in combination. The signal of the reporter molecule is amplified by continuous cleavage of the reporter molecule from the sensor molecule, the amplified signal is detected, and thus, the presence of a trace amount of the target nucleic acid can be detected. In the case of the nucleic acid probe, however, false positive reaction occurs, that is, the reporter molecule is cleaved by the sensor molecule before hybridization with the target nucleic acid. In addition, the sensor molecule misidentifies a nucleic acid having a base sequence close to that of a target nucleic acid as the target nucleic acid and hybridizes to it, which causes inadequate selectivity for a target nucleic acid. That is, this method has problems that in addition to troublesome operation, sensitivity and accuracy are not enough, false detection, etc. occur, and reliability is low.

In order to realize the tailor-made medicine, development of a technique is essential which can diagnose the influence (expression and repression of gene) of an administered drug on live cells quantitatively and quickly. The present inventors developed a nucleic acid probe, "QUAL probe", which has a very high selectivity for a target nucleic acid, does not require enzyme, reagent, etc., can be introduced into live cells, undergoes a chemical reaction on DNA or RNA, which can be a template in cells, can generate a signal accompanied by the progress of reaction, and enabled detection or sequence analysis, in cells, of DNA or RNA, which is expressed in cells (See Non-Patent Literature 1). In the case where this "QUAL probe" is used, however, there is problem that while detection of rRNA, etc., which are present in large amount, is easy, detection of mRNA, etc., present only in very small amounts, is very difficult, because only one signal is generated from one molecule of the RNA or DNA.

Non-Patent Literature 1

Imaging of RNA in Bacteria with Self-Ligating Quenched Probes sando S et al. J. AM. CHEM Soc. 2002, 124,9686

### Disclosure of Invention

The problem of the invention is to solve conventional problems, to develop a technique which can analyze viruses (target nucleic acids), present only in trace amounts, e.g. in early phase of virus infection, highly sensitively and highly quickly, a technique which can diagnose the influence (expression and repression of gene) on live cells quantitatively and quickly, and a technique which enables signal amplification, which the nucleic acid probe, "QUAL probe", could not achieve, etc., and to attain the following objects.

Accordingly, an object of the invention is to provide a nucleic acid probe which has a very high selectivity for a target nucleic acid, which can detect or analyze a trace amount of target nucleic acid highly sensitively, highly accurately, and highly quickly, besides easily by amplifying a signal generated from the nucleic acid probe hybridized to the target nucleic acid, which can measure the presence or behavior of the target nucleic acid even if it is in very small amounts and has a short life-span, etc., and which is suitable for a gene diagnosis, test for the presence of food-poisoning bacteria, diagnosis of tooth decay or periodontal disease, blood test, etc.

Another object of the invention is to provide a nucleic acid chip which can a trace amount of target nucleic acid highly sensitively by immobilizing the nucleic acid probe on a support, and which is suitable for a gene diagnosis, test for the presence of food-poisoning bacteria, diagnosis of tooth decay or periodontal disease, blood test, etc.

A further object of the invention is to provide a method for detecting a target nucleic acid and an apparatus for detecting a target nucleic acid which can detect or analyze a trace amount of target nucleic acid highly sensitively, highly accurately, and highly quickly by using the nucleic acid probe, and which are suitable for a gene diagnosis, test for the presence of food-poisoning bacteria, diagnosis of tooth decay or periodontal disease, blood test, etc.

Yet another object of the invention is to provide a method for screening a drug which can analyze an effect of administration of a drug by using the nucleic acid probe, and which can screen a desired drug efficiently.

Another object of the invention is to provide a gene diagnosis method which can diagnose the presence of a gene related to a specific disease highly efficiently and highly accurately by using the nucleic acid probe.

### - Nucleic acid probe -

The nucleic acid probe of the invention includes the following first to sixth nucleic acid probes.

The first nucleic acid probe is a nucleic acid probe for detecting a target nucleic acid and is designed so that the nucleic acid probe undergoes a conformational change after hybridization to the target nucleic acid, the nucleic acid probe, which underwent the conformational change, has a decreased binding strength and dissociates from the target nucleic acid. The first nucleic acid probe undergoes a conformational change upon hybridization to the target nucleic acid. As a result, the nucleic acid probe dissociates from the target nucleic acid, generating a signal, e.g. emission.

The second nucleic acid probe is a nucleic acid probe for detecting a target nucleic acid and includes a complementary region having a sequence complementary to at least a portion of the base sequence of the target nucleic acid, and a nucleic acid enzyme forming region capable of forming a self-cleaving nucleic acid enzyme. When the complementary region hybridizes to the target nucleic acid, the second nucleic acid probe undergoes a conformational change and the nucleic acid enzyme forming region can form the self-cleaving nucleic acid enzyme. As a result, the nucleic acid probe is cleaved, etc. by the self-cleaving nucleic acid enzyme, dissociates from the target nucleic acid, resulting in, for example, generation of a signal, e.g. emission.

The third nucleic acid probe can hybridize to a target nucleic acid and includes a conformation variable portion, of which conformation is capable of changing upon hybridization to the target nucleic acid, wherein the nucleic acid probe is capable of dissociating from the target nucleic acid when the conformation of the conformation variable portion has changed. When the third nucleic acid probe has hybridized to the target nucleic acid, the conformation of the conformation variable portion changes. As a result, the nucleic acid probe is cleaved, etc., dissociates from the target nucleic acid, resulting in, for example, generation of a signal, e.g. emission.

The fourth nucleic acid probe can hybridize to a target nucleic acid and includes a cleavage portion, which is cleaved upon hybridization to the target nucleic acid, wherein the nucleic acid probe is capable of forming a cleavage active region having cleavage activity toward the cleavage portion upon hybridization to the target nucleic acid. The fourth nucleic acid probe forms the cleavage active region upon hybridization to the target nucleic acid, and the cleavage portion is cleaved. As a result, the nucleic acid probe is cleaved, etc., dissociates from the target nucleic acid, resulting in, for example, generation of a signal, e.g. emission.

The fifth nucleic acid probe can hybridize to a target nucleic acid and includes a cleavage portion, and a signal-generating portion from which a signal is generated, wherein the cleavage portion is cleaved upon hybridization to the target nucleic acid and generates a first probe fragment and a second probe fragment which are capable of dissociating from the target nucleic acid. Before the fifth nucleic acid probe hybridizes to the target nucleic acid, a signal (e.g. emission) is not generated from the signal-generating portion. A signal (e.g. emission) is generated from the signal-generating portion upon hybridization to the target nucleic acid. As a result, the presence of the target nucleic acid is detected by detecting the signal (e.g. emission). The fifth nucleic acid probe, too, hybridizes to the target nucleic acid one after another, generating the signal (e.g. emission), and the signal is amplified in a short time (a large number of signals (e.g. emission) are generated from one target nucleic acid). Thus, even if the amount of the target nucleic acid is very small, the presence thereof is detected highly sensitively, highly accurately, and highly quickly.

The sixth nucleic acid probe can hybridize to a target nucleic acid and includes a cleavage portion, which is cleaved upon hybridization to the target nucleic acid, wherein the nucleic acid probe has formed a locked structure as a result of intramolecular hybridization of portions of the nucleic acid probe to each other until the nucleic acid probe hybridizes to the target nucleic acid. While the sixth nucleic acid probe has formed as a result of intramolecular hybridization of portions of the nucleic acid probe to each other before hybridization to the target nucleic acid, upon hybridization to the target nucleic acid, the complementary binding of the parts hybridizing to each other in the locked structure is unbound. This makes it easier for the cleavage portion to be cleaved, leading to cleavage of the cleavage portion, and a signal, e.g. emission, is generated.

The first to sixth nucleic acid probes hybridize to the target nucleic acid one after another, every time the probe hybridizes to the target nucleic acid, it undergoes a conformational change and is cleaved, generating the signal (e.g. emission), and the signal is amplified in a short time (a large number of signals (e.g. emission) are generated from one target nucleic acid). Thus, even if the amount of the target nucleic acid is very small, the presence thereof is detected highly sensitively, highly accurately, and highly quickly.

### - Nucleic acid chip -

The nucleic acid chip of the invention includes a support, and the nucleic acid probe of the invention immobilized on the support. While the nucleic acid probe fixed to the nucleic acid chip does not generate the signal before hybridization to the target nucleic acid, upon hybridization to the target nucleic acid, a signal (e.g. emission) is generated from the signal-generating portion. As a result, the presence or absence of the target nucleic acid is detected, for example, by detecting the presence or absence of the signal (e.g. emission). The nucleic acid chip of the invention allows the detection of the presence of the target nucleic acid highly sensitively, highly accurately, and highly quickly, even if the amount thereof is very small.

### - Method for detecting target nucleic acid -

The method for detecting a target nucleic acid of the invention includes the following first to third methods for detecting a target nucleic acid.

The first method for detecting a target nucleic acid is a method for detecting a nucleic acid in which a target nucleic acid is detected using a nucleic acid probe, the nucleic acid probe is designed such that it undergoes a conformational change upon hybridization to the target nucleic acid, the nucleic acid probe, which underwent the conformational change, has a decreased binding strength of hybridization and dissociates from the target nucleic acid.

The second method for detecting a target nucleic acid is a method for detecting a target nucleic acid using a nucleic acid probe including: a complementary region having a sequence complementary to at least a portion of the base sequence of the target nucleic acid; and a nucleic acid enzyme forming region capable of forming a self-cleaving nucleic acid enzyme. The second method for detecting a target nucleic acid includes: a hybridization step in which the nucleic acid probe is allowed to bind to a target nucleic acid complementarily; a dissociation step in which the self-cleaving nucleic acid enzyme is formed after the formation of hybridization, the nucleic acid probe undergoes a conformational change, as a result, the binding strength of hybridization decreases, and the nucleic acid probe dissociates from the target nucleic acid; and a detection step in which at least a part of the nucleic acid probe dissociated in the dissociation step is detected. In the method for detecting a target nucleic acid, the nucleic acid probe is allowed to bind to the target nucleic acid complementarily in the hybridization formation step. In the dissociation step, the self-cleaving nucleic acid enzyme is formed after the formation of hybridization, the nucleic acid probe undergoes a conformational change, as a result, the binding strength of hybridization decreases, and the nucleic acid probe dissociates from the target nucleic acid. In the detection step, at least a part of the nucleic acid probe dissociated in the dissociation step is detected.

The third method for detecting a target nucleic acid includes: a hybridization step in which the nucleic acid probe of the invention is allowed to hybridize to a target nucleic acid; and a target nucleic acid detection step in which the presence of the target nucleic acid is detected by detecting a signal generated from the nucleic acid probe upon hybridization to the target nucleic acid. In the method for detecting a target nucleic acid, in the hybridization step, the nucleic acid probe of the invention hybridizes to the target nucleic acid. In the target nucleic acid detection step, the presence or absence of the target nucleic acid is detected by detecting the presence or absence of the signal generated from the nucleic acid probe upon hybridization to the target nucleic acid.

A first probe fragment and a second probe fragment are generated by the cleavage of the nucleic acid probe upon hybridization to the target nucleic acid. In the case where the target nucleic acid detection step is such that the presence or absence of the target nucleic acid is detected by detecting the presence or absence of the emission of a light-emitting portion which is present in the first probe fragment, in the target nucleic acid detection step, the cleavage portion of the nucleic acid probe is cleaved upon hybridization, is divided into the first and second probe fragments, and the first and second probe fragments dissociate from the target nucleic acid. As a result, the quenching portion and light-emitting portion are separated from each other, and by detecting the emission generated by the light-emitting portion, which is present in the first probe fragment and has not been subjected to the action of the quenching portion, the presence or absence of the target nucleic acid is detected.

### - Method for screening drug -

The method for screening a drug of the invention includes a hybridization step in which the nucleic acid probe of the invention is allowed to hybridize to a target nucleic acid expressed due to administration of the drug, and a target nucleic acid detection step in which the presence or absence of the target nucleic acid is detected by detecting the presence or absence of a signal generated from the nucleic acid probe upon hybridization to the target nucleic acid, wherein the drug is screened by the presence or absence of the target nucleic acid.

In the hybridization step of the method for screening a drug, in the case where the target nucleic acid is expressed due to administration of the drug, the nucleic acid probe of the invention hybridizes to the target nucleic acid, however, in the case where the target nucleic acid is not expressed due to administration of the drug, the nucleic acid probe of the invention does not hybridize to the target nucleic acid. In the case where the target nucleic acid is expressed, in the target nucleic acid detection step, the presence of the target nucleic acid is detected by detecting the signal generated from the nucleic acid probe resulting from hybridization to the target nucleic acid. Based on this presence or absence of expression of target nucleic acid, a desired drug is screened.

A first probe fragment and a second probe fragment are generated by the cleavage of the nucleic acid probe after hybridization to the target nucleic acid. In the case where the target nucleic acid detection step is such that the presence or absence of the target nucleic acid is detected by detecting the presence or absence of the emission of a light-emitting portion which is present in the first probe fragment, in the target nucleic acid detection step, the cleavage portion of the nucleic acid probe is cleaved after hybridization to the target nucleic acid, is divided into the first and second probe fragments, and at least one of the first and second probe fragments dissociates from the target nucleic acid. As a result, the presence or absence of expression of the target nucleic acid due to administration of the drug is detected, and based on this presence or absence of expression of target nucleic acid, a desired drug is screened.

### - Apparatus for detecting a target nucleic acid -

The apparatus for detecting a target nucleic acid of the invention include the following first and second apparatuses.

The first apparatus for detecting a target nucleic acid is a device for analyzing a nucleic acid in which a nucleic acid probe for detecting a target nucleic acid in a sample is immobilized on a support, wherein the nucleic acid probe is designed so that the nucleic acid probe undergoes a conformational change upon hybridization to the target nucleic acid, the nucleic acid probe, which underwent a conformational change, has a decreased binding strength of hybridization and dissociates from the target nucleic acid.

The second apparatus for detecting a target nucleic acid includes a unit configured to allow the nucleic acid probe of the invention to hybridize to the target nucleic acid; and a target nucleic acid detector configured to detect the presence of the target nucleic acid by detecting a signal generated from the nucleic acid probe upon hybridization to the target nucleic acid. Preferably, the second apparatus for detecting a target nucleic acid is one of the first and second aspects. In the first aspect, the second apparatus for detecting a target nucleic acid includes a database part which stores database of genomic information; and a data analysis part which compares and analyzes data of the result of detection by the target nucleic acid detector and genomic information data stored in the database part. In the second aspect, the second apparatus for detecting a target nucleic acid includes a communication part capable of communicating with internet, and capable of accessing to database of genomic information on the internet; and a data analysis part which compares and analyzes data of the result of detection by the target nucleic acid detector and genomic information data of the database of genomic information on the internet. More preferably, the database of genomic information includes genomic information of a healthy individual and genomic information of a patient, related to a particular disease.

In the apparatus for detecting a target nucleic acid, the unit allows the nucleic acid probe of the invention to hybridize to the target nucleic acid. By the target nucleic acid detector, the signal generated from the nucleic acid probe upon hybridization to the target nucleic acid is detected, and thereby the presence of the target nucleic acid is detected.

A first probe fragment and a second probe fragment are generated by the cleavage of the nucleic acid probe upon hybridization to the target nucleic acid. In the case where the target nucleic acid detector is such that the presence or absence of the target nucleic acid is detected by detecting the presence or absence of the emission of a light-emitting portion which is present in the first probe fragment, the cleavage portion of the nucleic acid probe is cleaved after hybridization to the target nucleic acid, is divided into the first and second probe fragments, and the first and second probe fragments dissociate from the target nucleic acid. As a result, the quenching portion and light-emitting portion are separated from each other, and the target nucleic acid detector detects the emission generated by the light-emitting portion, which is present in the first probe fragment and has not been subjected to the action of the quenching portion. As a result, the presence of the target nucleic acid is detected.

The gene diagnosis method of the invention uses the apparatus for detecting a target gene of the invention, and includes a target nucleic acid expression level quantitative determination step in which the expression level of a target nucleic acid related to a specific disease in a subject is determined quantitatively, and a diagnosis step in which the expression level of the target nucleic acid related to the specific disease in a subject, and the expression levels, in a healthy individual and in a patient, of the target nucleic acid related to the specific disease which are contained in a database of genomic information, are compared by a data analysis part to diagnose whether or not the subject is a patient of the specific disease.

The gene diagnosis method of the invention uses the apparatus for detecting a target gene of the invention, and includes a target nucleic acid expression level quantitative determination step in which the expression level of a target nucleic acid related to a specific disease in a subject is determined quantitatively, and a diagnosis step in which the expression level of the target nucleic acid related to the specific disease in a subject, and the expression levels, in a healthy individual and in a patient, of the target nucleic acid related to the specific disease which are contained in a database of genomic information, are compared by a data analysis part to diagnose whether or not the subject is a patient of the specific disease.

In the gene diagnosis method, in the target nucleic acid expression level quantitative determination step, the expression level of a target nucleic acid related to a specific disease in a subject is determined quantitatively using the apparatus for detecting a target gene of the invention. In the diagnosis step, the expression level of the target nucleic acid related to the specific disease in the subject, and the expression levels, in a healthy individual and in a patient, of the target nucleic acid related to the specific disease which are contained in the database of genomic information, are compared by the data analysis part to diagnose whether or not the subject is a patient of the specific disease.

### Brief Description of Drawings

FIG. 1 is a schematic diagram showing an example of a process of the method for detecting a target nucleic acid of the invention using the nucleic acid probe of the invention.
FIG. 2 is a schematic illustration showing an example of the nucleic acid probe of the invention (Example 1).
FIG. 3 is a schematic view showing a state where the nucleic acid probe of the invention (Example 1) has hybridized to a target nucleic acid.
FIG. 4 is a graph showing the result of Example 1 in which the target nucleic acid method of the invention using the nucleic acid probe of the invention was carried out.
FIG. 5A is a schematic diagram showing an example of the nucleic acid probe of the invention which adopts a stem loop structure before hybridization to a target nucleic acid (right view), and a state where the nucleic acid probe has hybridized to a target nucleic acid (left view).
FIG. 5B is a schematic view showing one-dimensional structure of the nucleic acid probe of FIG. 5A.
FIG. 6 is a schematic diagram showing an example of a process (amplification of emission) of the method for detecting a target nucleic acid of the invention using the nucleic acid probe of the invention, which adopts a stem loop structure.
FIG. 7 is a schematic diagram showing an example of the method for screening a drug of the invention using the nucleic acid probe of the invention.
FIG. 8 is a schematic diagram showing another example of the method for screening a drug of the invention using the nucleic acid probe of the invention.
FIG. 9 is a block diagram showing an example of the apparatus for detecting a target nucleic acid of the invention using the nucleic acid probe of the invention.
FIG. 10 is a picture data representing the result (detection result of emission) of Example 3 in which the method for detecting a target nucleic acid of the invention using the nucleic acid probe of the invention was carried out.
FIG. 11 is a picture data representing the result of Example 5 in which the method for detecting a target nucleic acid of the invention using the nucleic acid probe of the invention was carried out.
FIG. 12 is a schematic diagram showing forms of hybridization in Example 6 in which the method for detecting a target nucleic acid of the invention using the nucleic acid probe of the invention was carried out.
FIG. 13 is a picture data representing the result of Example 6 in which the method for detecting a target nucleic acid of the invention using the nucleic acid probe of the invention was carried out.

### Best Mode for Carrying Out the Invention

### (Nucleic acid probe)

The nucleic acid probe of the invention can hybridize to a target nucleic acid, the nucleic acid probe is preferably the above-mentioned first to sixth nucleic acid probes, more preferably those having two characteristics of the first to sixth nucleic acid probes, particularly preferably those having three characteristics. The nucleic acid probe of the invention is not particularly limited as long as it has a characteristic of the first to sixth nucleic acid probes, and may comprise other parts appropriately selected according to the purpose.

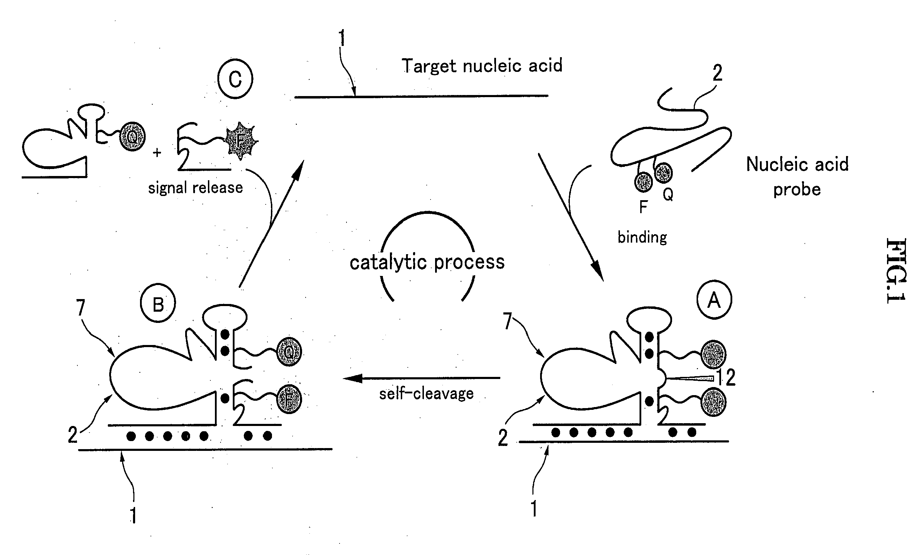

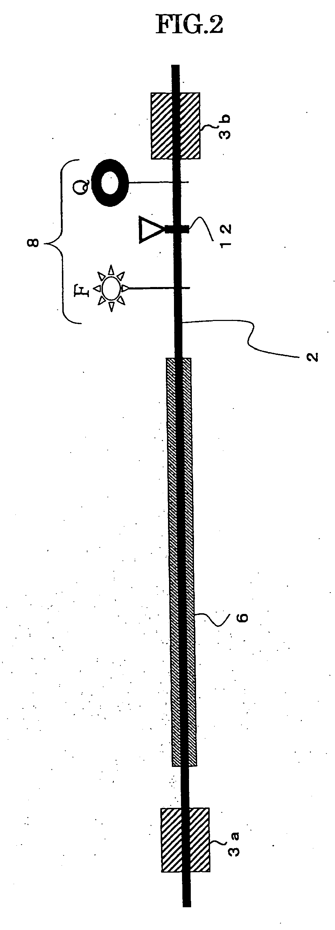

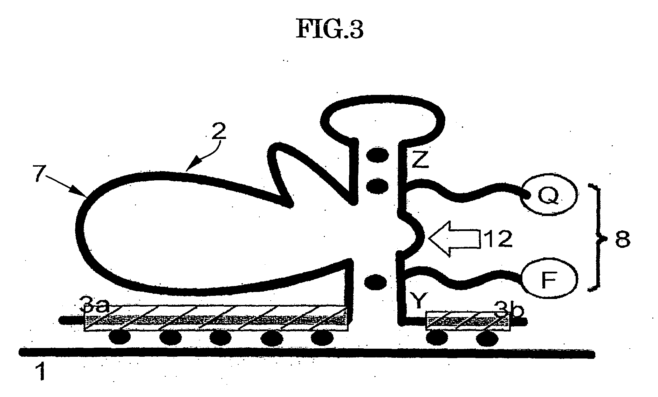

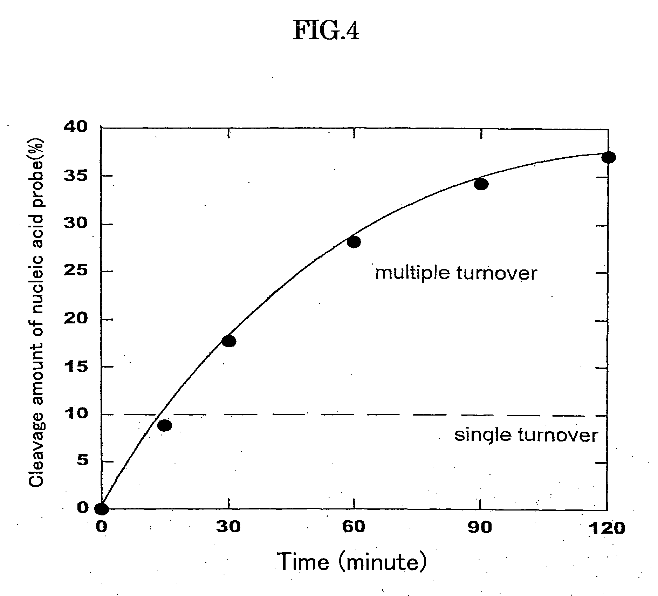

The nucleic acid probe of the invention will be described below with reference to drawings. FIG. 1 is a schematic diagram showing an example of reaction cycle in the method for detecting a nucleic acid, FIG. 2 is a schematic view showing an example of the nucleic acid probe of the invention, FIG. 3 is a schematic view showing a state where the nucleic acid probe of the invention has hybridized to a target nucleic acid, and FIG. 4 is a graph showing the result of detection reaction of a target nucleic acid according to one example of the invention.

The first nucleic acid probe is designed so that it undergoes a conformational change upon hybridization to the target nucleic acid, the nucleic acid probe, which underwent the conformational change, has a decreased binding strength and dissociates from the target nucleic acid.

This configuration allows the amplification of the signal representing the presence of a target nucleic acid, improving detection sensitivity.

For example, as shown in FIG. 1, a cycle including binding or dissociation of a nucleic acid probe is formed on a molecule of target nucleic acid 1. In FIG. 1, when a nucleic acid probe 2 and the target nucleic acid 1 come into contact with each other, they hybridize to each other, forming a complex (A). The hybridized nucleic acid probe 2 forms a self-cleaving nucleic acid enzyme 7, and the self-cleaving nucleic acid enzyme 7 cleaves the probe at a cleavage site 12 having a specific sequence (B). The cleaved nucleic acid probe cannot keep a binding state with the target nucleic acid 1 and spontaneously dissociates (C). At this time, a fluorescent substance F loses interaction with a quenching substance Q and emits fluorescence. To the free target nucleic acid 1, a new nucleic acid probe 2 hybridizes again, and by repeating of the processes (A) to (C), fluorescence signal is amplified. In this way, since amplification of detection signal is carried out on one molecule of target nucleic acid, the target nucleic acid 1 can be detected even if it is present in very small amounts or has a short life-span.

Further, since restriction enzyme, chemical substance, etc. are not used in this reaction cycle, it is possible to introduce the nucleic acid probe of the invention directly into living cells to allow the processes (A) to (C) to proceed. Therefore, the invention allows "recognition", "signal amplification" and "diagnosis", in cells, of target nucleic acid such as gene, for example, enabling a screening in which a relationship between an administered drug and the resulting expression or repression of gene is directly observed.

Further, since there is no need for gene amplification by, e.g. PCR, control of reaction temperature, etc., particular equipments or facilities such as a laboratory and analyzer are not required, and thus even at the places other than medical or research facilities, such as home, school, workplace, etc., gene diagnosis can be carried out.

Here, "target nucleic acid" mentioned in the invention means a nucleic acid or gene, which is intended for quantitative detection, qualitative detection, or simple detection, and includes any state of purified and unpurified one. The target nucleic acid may be any kind of nucleic acids, including RNA, DNA, PNA, artificially modified nucleic acid, and the like. The base sequence of target nucleic acid can be determined by, for example, Maxam-Gilbert method and dideoxy method.

Preferably, the conformational change of the nucleic acid probe of the invention comprises formation of a self-cleaving nucleic acid enzyme. This enables easy formation of cycle consisting of binding or dissociation of nucleic acid probe on one molecule of target nucleic acid, improving the detection sensitivity of target nucleic acid. In addition, restriction enzyme, etc. are not used in order to cleave a nucleic acid probe, thus, only by introducing the nucleic acid probe of the invention into cells, target nucleic acid in cells can be easily detected utilizing a specific enzymatic reaction.

Further, preferably, the nucleic acid probe of the invention is one comprising a labeling substance. This allows easy confirmation of the presence of target nucleic acid. Furthermore, more preferably, such labeling substance is such that it emits different signals before and after the nucleic acid probe dissociates from the target nucleic acid. Details of the labeling substance will be described later.

The second nucleic acid probe, as shown in FIGS. 2 and 3, comprises complementary regions 3a and 3b, and an nucleic acid enzyme forming region 6 capable of forming a self-cleaving nucleic acid enzyme. The complementary regions have a sequence complementary to at least a portion of the base sequence of target nucleic acid.

The third nucleic acid probe, as shown in FIGS. 2 and 3, comprises a cleavage portion 12, as a conformation variable portion, of which conformation can change upon hybridization to the target nucleic acid, and can dissociate from the target nucleic acid when the conformation of the conformation variable portion has changed, i.e., when the cleavage portion 12 has been cleaved.

The fourth nucleic acid probe, as shown in FIGS. 2 and 3, comprises a cleavage portion 12, which is cleaved upon hybridization to the target nucleic acid, and can form a cleavage active region 7 having cleavage activity toward the cleavage portion 12 upon hybridization to the target nucleic acid.

The fifth nucleic acid probe, as shown in FIGS. 2 and 3, comprises a cleavage portion 12 and signal-generating portion 8 from which a signal is generated. The cleavage portion 12 generates first and second probe fragments, which, upon hybridization to the target nucleic acid, are cleaved and capable of dissociating from the target nucleic acid.

FIG. 2 shows a state where a nucleic acid probe is stretched as a single strand. FIG. 3 shows a state where a nucleic acid probe has hybridized to a target nucleic acid. As shown in FIGS. 2 and 3, a nucleic acid probe 2 can hybridize to a target nucleic acid, and comprises base sequences 3a and 3b, a nucleic acid enzyme forming region 6, a light-emitting substance F as the light-emitting portion included in a signal-generating portion 8, a cleavage portion 12, and a quenching substance Q as the quenching portion included in the signal-generating portion.

The base sequences 3a and 3b are regions which can hybridize to the target nucleic acid 1, and are located at both ends of the nucleic acid probe 2.

The nucleic acid enzyme forming region 6 shown in FIG. 2 forms the cleavage active region 7 when the nucleic acid probe 2 has hybridized to the target nucleic acid 1, and exhibits cleavage activity toward the cleavage portion 12 (FIG. 3). At this time, in the nucleic acid probe 2, portions thereof have hybridized within the molecule, specifically, portions of the regions in both sides of the cleavage portion 12 have hybridized, respectively, with the cleavage portion 12 centered (via the cleavage portion 12) (Y and Z in FIG. 3).

The cleavage portion 12 is composed of ribonucleic acid (sugar in nucleotide is ribose) and can be cleaved by a ribozyme activity which the cleavage active region 7 exhibits.

As shown in FIGS. 2 and 3, the light-emitting substance F and quenching substance Q are located adjacent to each other with the cleavage portion 12 centered (via the cleavage portion 12).

This enables each of the second to fifth nucleic acid probes to function as, for example, a self-cleavage type gene diagnosis probe, by making the target nucleic acid act as an allosteric effector. Each fragment of self-cleaved nucleotide cannot maintain a complementary binding (hybridization) with the target nucleic acid under isothermal conditions, resulting in spontaneous dissociation. Thereafter, a new nucleic acid probe hybridizes to the target nucleic acid, forms self-cleaving nucleic acid enzyme, and then dissociates. Thus, by a sequential repeat of catalytic cycle as shown in FIG. 1, a detection signal, typically a fluorescence signal, is amplified, which enabled to detect easily a trace amount of nucleic acid, gene, etc., which was very difficult to detect by a conventional technique of measuring a nucleic acid, without, for example, nucleic-acid amplification such as PCR. In addition, addition of a special enzyme or reagent is not required for each of the second to fifth nucleic acid probes to form a self-cleaving nucleic acid enzyme, enabling introduction into living cells. Therefore, by using each of the second to fifth nucleic acid probes, even behavior of nucleic acid or gene at one molecular level in cells can be observed.

In other words, the nucleic acid probe 2 comprises complementary base sequences 3a and 3b, which can hybridize to the target nucleic acid 1, at both ends thereof (See FIGS. 2 and 3). Thus, as shown in FIG. 1, in the presence of the target nucleic acid 1, when base sequences 3a and 3b hybridize to the nucleic acid sequence 1 ("A" in FIG. 1), nucleic acid enzyme forming region 6 adopts a three dimensional conformation, and thus the cleavage active region 7 is formed, yielding cleavage activity (ribozyme activity) toward the cleavage portion 12. The cleavage portion 12 (ribose portion) is cleaved (self-cleaved), divided into the first probe fragment comprising the light-emitting substance F and the second probe fragment comprising the quenching substance Q ("B" in FIG. 1), and each of the probe fragments is dissociated from the target nucleic acid 1. As a result, the light-emitting substance F and quenching substance Q, which had located adjacent to each other, are located apart from each other ("C" in FIG. 1). When the light-emitting substance F and quenching substance Q are located adjacent to each other, emission of the light-emitting substance F is in a quenched state 8 by the action of the quenching substance Q through fluorescence resource energy transfer (FRET) between them; however, the fluorescence resource energy transfer is lost, and quenching effect of the quenching substance Q does not occur, resulting in that the light-emitting substance F emits light ("C" in FIG. 1). This emission is generated every time the nucleic acid probe hybridizes to the target nucleic acid 1, and is amplified through the repeat of this reaction (catalytic process; cycle of A to C in FIG. 1). Namely, when the nucleic acid probe 2 is cleaved, the first and second probe fragments cannot keep hybridization and dissociates from the target nucleic acid 1. Then, the target nucleic acid 1 becomes free, and thus a new nucleic acid probe 2 hybridizes, leading to the repeat of the cycle of A to C in FIG. 1. The nucleic acid probe 2 can make the target nucleic acid act as an allosteric effector. Therefore, even if the target nucleic acid 1 is present in very small amounts (< 10 ng/µl) or has a short life-span, the presence thereof can be detected easily, for example, with eyes by amplifying the light emitted by the nucleic acid probe.

In this case, there is no need for a nucleic-acid amplification as carried out previously using PCR method, etc., and very small amounts of nucleic acid, which was unable to measure before, can be detected highly sensitively, highly accurately, and highly quickly. Further, since restriction enzymes, chemical substances, or the like are not used, the nucleic acid probe 2 can be introduced directly into live cells, and is allowed to hybridize, e.g. to virus. Therefore, by the nucleic acid probe 2, very small amounts of target nucleic acid present in cells can be detected.

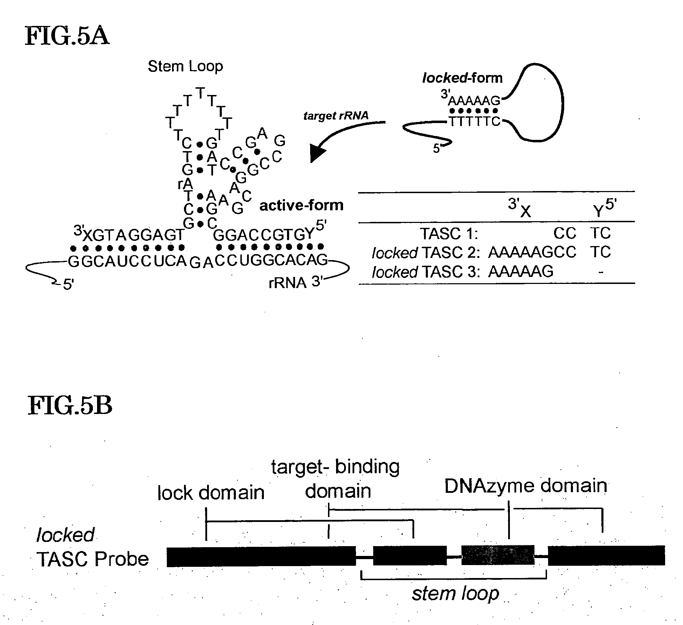

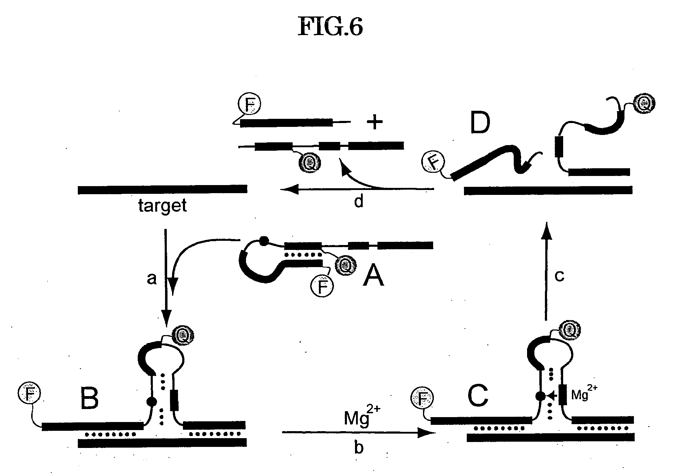

FIGS. 5A and 5B, and FIG. 6 are schematic illustrations showing an example of the sixth nucleic acid probe. The sixth nucleic acid probe, as shown in FIGS. 5A and 5B, and FIG. 6, can hybridize to a target nucleic acid, comprises a cleavage portion (rA), which is cleaved when hybridized to the target nucleic acid, and parts of nucleic acid probe hybridize to each other intramolecularly to form a locked structure (stem loop structure) to until the probe hybridizes to the target nucleic acid.

More specifically, this nucleic acid probe comprises: two base sequences (5' XGTAGGAGT 3' at the 5' end, and 3' YGTGCCAGG 5' at the 3' end), which can hybridize with the target nucleic acid; cleavage active region; a light-emitting substance F as the light-emitting portion included in the signal-generating portion; a cleavage portion (rA); and a quenching substance Q as the quenching portion included in the signal-generating portion, and the nucleic acid probe has a locked structure (stem loop structure) in which adenine (A) oligomer and thymine (T) oligomer hybridize to each other.

This nucleic acid probe, as shown in FIGS. 5A and 5B, and FIG. 6, forms the locked structure (stem loop structure) before hybridization to the target nucleic acid. The adenine (A) oligomer is located at the furthest end in the 5' end side of the nucleic acid probe, and the thymine (T) oligomer is located in the 3' end side but not at the furthest end. Thus, as shown in FIGS. 5A and 5B, and FIG. 6, the locked structure adopts a stem loop structure. In the nucleic acid probe adopting the stem loop structure, nucleotides in the 3' end side extend beyond the stem. While the nucleic acid probe has this stem loop structure (before hybridization to the target nucleic acid), it has not formed the cleavage active region, and cleavage at the cleavage portion (rA: structure where base is adenine and sugar is ribose) does not occur. Therefore, before the nucleic acid probe hybridizes to the target nucleic acid, in the nucleic acid probe, it does not happen that the cleavage active region is formed and cleaves the cleavage portion, and there is no probability of e.g. false detection.

In the invention, the nucleic acid probe of the invention, which has such locked structure (stem loop structure) and does not exhibit cleavage activity toward the cleavage portion before hybridization to the target nucleic acid, may be referred to as "Locked TASC" probe. In contrast, such nucleic acid probe of the invention that does not have the stem loop structure as described above may be referred to as "TASC" probe.

In the case of this nucleic acid probe, i.e., the "Locked TASC" probe, as shown in FIGS. 5B and 6, the probe cannot form a self-cleaving nucleic acid enzyme when it adopts the locked structure (stem loop structure), and thus does not form the cleavage active region. Conversely, while the nucleic acid probe forms the cleavage active region, it does not adopt the locked structure (stem loop structure). Between the form or structure in which the locked structure (stem loop structure) is adopted, and the form or structure in which the cleavage active region is formed, it is necessary for the nucleic acid probe to undergo a large change in form or conformation. In the case of the "Locked TASC" probe, this change in form or conformation easily occurs due to hybridization of the nucleic acid probe to the target nucleic acid. That is, the sixth nucleic acid probe is designed such that the affinity (binding strength, hybridization strength) of two base sequences capable of hybridizing to the target nucleic acid, to the target nucleic acid is larger (stronger) than the affinity (binding strength, hybridization strength) in the locked structure (stem loop structure). Therefore, the nucleic acid probe adopts the locked structure (stem loop structure) before hybridization to the target nucleic acid. However, when the target nucleic acid is present, the two base sequences capable of hybridizing to the target nucleic acid interact with the complementary region in the target nucleic acid. At this time, since the affinity (binding strength, hybridization strength) of the two base sequences to the target nucleic acid is large (strong), the locked structure (stem loop structure) is easily dissociated. As a result, the nucleic acid probe undergoes a large change in form or conformation, which allows for hybridization to the target nucleic acid.

In the nucleic acid probe, the affinity (binding strength, hybridization strength) of the two base sequences capable of hybridizing with the target nucleic acid, to the target nucleic acid (hereinafter referred to as "A2"), and the affinity (binding strength, hybridization strength) in the locked structure (stem loop structure) (hereinafter referred to as "A1") can be appropriately adjusted by, for example, the number of bases in the base sequence capable of hybridizing to the target nucleic acid or the number of bases in the base sequence of a complementary strand in the locked structure, and the number of hydrogen bond between each base pair (2 or 3) during hybridization.

A2 > A1, in the relationship between the A1 and A2, can be achieved, for example, by making the number of bases in the two base sequences capable of hybridizing with the target nucleic acid more than the number of bases in the base sequence of a complementary strand in the locked structure. In the example shown in FIG. 5A, the number of the A2 is "9 bases × 2 places = 18". In contrast, the number of the A1 is "5", and the number of the A2 is more than 3 times to less than 4 times as many as that of the A1.

As shown in FIGS. 5A and 6, in the nucleic acid probe, the one-dimensional cleavage active region as shown in FIG. 5B forms the cleavage active region when the nucleic acid probe has hybridized to the target nucleic acid, and exhibits cleavage activity toward the cleavage portion (site displayed by rA in FIG. 5A, by Q in FIG. 6). At this time, in the nucleic acid probe, portions thereof have hybridized within the molecule, specifically, portions of the regions in both sides of the cleavage portion have hybridized, respectively, with the cleavage portion centered (via the cleavage portion) (both sides of rA in FIG. 5A, both sides of the site displayed by Q in B and C in FIG. 6).

The cleavage portion is composed of ribonucleic acid (sugar in nucleotide is ribose) and can be cleaved by a ribozyme activity which the cleavage active region exhibits.

As shown in A of FIG. 6, the light-emitting substance F and quenching substance Q are located adjacent to each other while the nucleic acid probe adopts the locked structure (stem loop structure), during which the emission of the light-emitting substance F is quenched by the action of the quenching substance Q.

As shown in the left figure in FIG. 5A and B in FIG. 6, the nucleic acid probe comprises two base sequences at both ends thereof, which are complementary to the target nucleic acid and are capable of hybridizing to the target nucleic acid in the presence of the target nucleic acid (target) (See FIG. 5A and B in FIG. 6). Thus, when the two base sequences hybridize to the nucleic acid (FIG. 5A and B in FIG. 6), the cleavage active region is formed, yielding cleavage activity (ribozyme activity) toward the cleavage portion (In the C in FIG. 6, magnesium ion is positioned in the center of the cleavage active region, thereby resulting in a state where cleavage activity is exhibited or improved). The cleavage portion (ribose portion) is cleaved (self-cleaved), divided into a first probe fragment comprising the light-emitting substance F and a second probe fragment comprising the quenching substance Q (D in FIG. 6), and each of the probe fragments is dissociated from the target nucleic acid. The light-emitting substance F and quenching substance Q which had located adjacent to each other, are located apart from each other when the nucleic acid probe has hybridized to the target nucleic acid (B in FIG. 6), and thus quenching effect of the quenching substance Q does not occur, resulting in that the light-emitting substance F emits light. The emission by the light-emitting substance F occurs every time the nucleic acid probe hybridizes to the target nucleic acid, and is amplified through the repeat of this reaction (cycle of A to D in FIG. 6). Namely, when the nucleic acid probe is cleaved, the first and second probe fragments cannot keep hybridization and dissociates from the target nucleic acid. Then, the target nucleic acid becomes free, and thus a new nucleic acid probe hybridizes, leading to the repeat of the cycle of A to D in FIG. 6. The nucleic acid probe can make the target nucleic acid act as an allosteric effector. Therefore, even if the target nucleic acid is present in very small amounts (< 10 ng/µl) or has a short life-span, the presence thereof can be detected easily, for example, with eyes by amplifying the light emitted by the nucleic acid probe.

The nucleic acid probe of the invention may be composed of nucleotide chain alone, or part thereof may be composed of nucleotide chain. In the latter case, the portion other than that composed of nucleotide may be composed of amino acid chain, sugar chain, or synthetic polymer chain, for example. Among these, the entire of the nucleic acid probe is preferably composed of nucleotide chain in terms of production efficiency, etc.

The method for producing the nucleotide chain in the nucleic acid probe is not particularly limited and can be appropriately selected according to the purpose. Examples thereof include a chemical synthesis method, microbial method in which plasmid vector, phage vector, or the like is used, mechanical synthesis method in which a nucleic-acid synthesizing machine is used, and the like. Among these, the nucleic-acid synthesizing machine is preferred in terms of excellent mass productivity.

The number of bases in the sequence (full length) of the nucleotide chain in the nucleic acid probe is not particularly limited and can be appropriately selected according to the purpose, for example, preferably 5 to 100, and more preferably 10 to 70.

When the number of bases in the sequence (full length) of the nucleotide chain exceeds 100, detection accuracy may decrease due to non-specific hybridization. In addition, the target nucleic acid may not be detected in cells efficiently due to the decrease of the permeability to cell membrane. When the number is less than 5, binding strength of hybridization becomes insufficient, which may cause the decrease of detection accuracy.

The nucleic acid probe of the invention can hybridize to the target nucleic acid and comprise a region (site) capable of forming complementary strands with the target nucleic acid. This allows the nucleic acid probe to capture the target nucleic acid to be detected in a sample.

As the number of region (site) capable of forming complementary strands with the target nucleic acid, at least one region is required, the number may be two or more, but preferably, two. When the number of region (site) capable of forming the complementary strands is plural, there are advantages, for example, in that the target nucleic acid is captured well, detection sensitivity can be improved, as well as the binding strength with the target nucleic acid can be controlled easily, and furthermore, the nucleic acid probe can easily form the cleavage active region described later stably and efficiently when the nucleic acid probe has hybridized to the target nucleic acid.

The position of the region (site) capable of forming complementary strands with the target nucleic acid is not particularly limited, can be appropriately selected according to the purpose, and may be any portion of the nucleic acid probe. For example, when the number of region (site) capable of forming the complementary strands with the target nucleic acid is two, as shown in FIG. 3, the position may be both ends of the nucleic acid probe (complementary parts 3a and 3b in FIG. 3) or may be one end and central part of the nucleic acid probe as shown in FIGS. 5A and 5B. Among these, in the latter case (in the case as shown in FIGS. 5A and 5B), the nucleic acid probe undergoes a large change in form or conformation before and after hybridization to the target nucleic acid, and thus, by the hybridization, the cleavage active region described later can be formed, allowing the cleavage activity toward a cleavage portion described later to be exhibited. Therefore, the latter case is advantageous in that there is no false cleavage, not causing problems such as a false detection.

The length of the complementary strand (region capable of hybridizing to the target nucleic acid), i.e., the number of bases in the sequence of one strand of the complementary strands is not particularly limited and can be appropriately selected according to the purpose, preferably 1 to 13, more preferably 2 to 10 in that selectivity to the target nucleic acid is high, particularly preferably 6 to 9. The binding strength with the target nucleic acid is approximately proportional to the number of bases in the sequence of the complementary strand. For example, in the example of A in FIG. 1, the binding strength of the nucleic acid probe 2 to the target nucleic acid 1 is sum of the binding strengths of complementary parts 3a and 3b. In the case where cleavage or the like has taken place to the nucleic acid probe 1 ("B" in FIG. 1), the binding strength in each nucleic acid probe fragment is the binding strength of the portion binding to the target nucleic acid complementarily (complementary region 3a or complementary region 3b) alone. Namely, by cleavage, the binding strength to the target nucleic acid 1 decreases to about half. By utilizing this decrease of binding strength, reaction cycle containing processes of hybridization to the target nucleic acid 1 and dissociation can be established.

When the number of bases in the sequence is 1 to 13, it is advantageous in that each complementary region cannot keep hybridization with the target nucleic acid by itself at the temperature close to internal body temperature of an organism (close to 37°C), allowing a spontaneous dissociation more easily and enabling easy establishment of reaction cycle of a nucleic acid probe and a target nucleic acid after introduction of the nucleic acid probe directly into living body or cells. When the number of bases in the sequence is 2 to 10, and furthermore 6 to 9, it is more preferable in that the effect is remarkable. The number of bases in the sequence of each complementary strand may be the same as each other or may be different.

### - Cleavage active region -

Preferably, the nucleic acid probe of the invention comprises, for example, the cleavage active region (e.g. nucleic acid enzyme forming region) 6 capable of forming a site having cleavage activity such as self-cleaving nucleic acid enzyme toward the cleavage portion. This causes the nucleic acid probe 2 to undergo a conformational change in which, for example, self-cleaving nucleic acid enzyme is formed after hybridization to the target nucleic acid 1, and then the nucleic acid probe 2 can dissociate from the target nucleic acid. It is preferable that the cleavage active region can be formed upon hybridization to the target nucleic acid.

The cleavage active region is not particularly limited and can be appropriately selected according to the purpose. Suitable examples include ones which exhibit cleavage activity under given conditions, for example, a DNA or RNA chain, which is capable of exhibiting cleavage activity (enzyme activity) e.g. DNAzyme activity, RNAzyme activity, and ribozyme activity when adopted a specific form or conformation.

The cleavage active region (nucleic acid enzyme forming region) includes a region which can undergo a conformational change, when the nucleic acid probe has bound to the target nucleic acid, so as to form a cavity in which a metal ion for exhibiting enzyme activity is captured. The cleavage active region has cleavage activity, e.g. enzyme activity, such as, for example a specific cleavage of nucleotide in the nucleic acid probe molecule. Since a restriction enzyme or the like is not required for the nucleic acid probe, only introduction of the nucleic acid probe into cells enables the detection of the target nucleic acid in cells utilizing a specific enzyme reaction. Normally, in the case where specific cleavage is required to occur, an enzyme recognizing a specific sequence site to be cleaved must be selected and used. When a nucleic acid is intended to be cleaved at any sequence, a restriction enzyme which recognizes a specific sequence site and cleaves it does not always exist in every case. The nucleic acid probe of the invention, however, has not only a nucleic acid enzyme forming region, but also a specific sequence which is, for example, recognized or cleaved by a self-cleaving nucleic acid enzyme within the same molecule. Thus, the nucleic acid probe can be arbitrarily designed so that it exhibits desired prosperities of enzyme activity and there is no limitation mentioned above as in the case where restriction enzyme is used.

It is preferable that the cleavage site 12 is arranged at the position at which cleavage activity is exhibited most efficiently when the self-cleaving nucleic acid enzyme 7 is formed after the nucleic acid probe has hybridized.

Preferably, the nucleic acid enzyme forming region 6 is located in the region between one complementary region 3a and other complementary region 3b when there are two or more complementary regions, for example. Such configuration allows a nucleic acid probe to be fixed steadily and allows the self-cleaving nucleic acid enzyme 7 to be more stable.

As a result of the action of the self-cleaving nucleic acid enzyme, it becomes easier to design the nucleic acid probe so that when a nucleotide is cleaved, the binding strength between the nucleic acid probe and the target nucleic acid decreases.

In the invention, self-cleaving nucleic acid enzyme, which is formed from the nucleic acid enzyme forming region, may have any activity of cleavage activity, binding activity, and other activities, but self-cleaving nucleic acid enzyme having the cleavage activity is preferred. For example, in the case where the nucleic acid probe is cleaved between two complementary regions by the action of self-cleaving nucleic acid enzyme after hybridization of the nucleic acid probe to the target nucleic acid, before cleavage, binding state with the target nucleic acid is maintained by the total strength of binding strengths of each complementary region, however, after cleavage, shortened fragments each cannot keep hybridization with the target nucleic acid, resulting in the dissociation from the target nucleic acid. Then, the target nucleic acid becomes free, which binds with a new nucleic acid probe, and reaction cycle, in which formation of self-cleaving nucleic acid enzyme, cleavage, dissociation, formation of a new hybridization, can be repeated sequentially. Therefore, when a nucleic acid probe is used which is designed so that a free cleaved fragment emits a signal, signal is amplified on one molecule of the target nucleic acid, and thus, detection sensitivity can be improved.

Examples of the self-cleaving nucleic acid enzyme which is formed from the nucleic acid enzyme forming region 6 include, for example, a DNA or RNA enzyme, and self-cleaving nucleic acid enzyme may be any of them. These nucleic acid enzymes may be any type of hammerhead type, hairpin type, HDV type, and other types.

In order to form the cleavage active region, it is preferable that the nucleic acid probe comprise a region capable of hybridizing intramolecularly upon hybridization to the target nucleic acid. This makes it easier for the nucleic acid probe to adopt a three-dimensional conformation by which the cleavage active region is formed. Further, this case is advantageous, for example, in that the three-dimensional conformation has an excellent stability and cleavage activity, etc. of the cleavage active region to be formed are excellent.

In the nucleic acid probe, the number of the region capable of hybridizing intramolecularly upon hybridization to the target nucleic acid is not particularly limited and can be appropriately selected according to the purpose. For example, 2 or more is preferable, which is advantageous in that the cleavage active region is easily formed.

In the nucleic acid probe, the position of the regions capable of hybridizing intramolecularly upon hybridization to the target nucleic acid is not particularly limited and can be appropriately selected according to the purpose. For example, the regions are preferably located adjacent to each other with the cleavage portion described later centered (via the cleavage portion), in addition to this position, the regions may be located in the side facing the cleavage portion which is the center of the regions mentioned above (between the regions) and is described later (in or near the cleavage active region described later). When the regions are located adjacent to each other with the cleavage portion centered (via the cleavage portion), it is advantageous in that the cleavage active region and cleavage portion can be faced so that the cleavage active region can cleave the cleavage portion. In addition, when the regions are present in the side facing the cleavage portion which is the center of the regions mentioned above (between the regions) and is described later (in or near the cleavage active region described later), it is advantageous, for example, in that a complex three-dimensional conformation can be provided to the cleavage active region, and the cleavage activity thereof can be enhanced.

In the nucleic acid probe, the number of bases in the sequence of regions capable of hybridizing intramolecularly upon hybridization to the target nucleic acid is not particularly limited and can be appropriately selected according to the purpose.

If the number of bases in the sequence of at least one of the Y and Z is less than 2, the cleavage active region may not be formed or structure thereof may not be maintained.

The total number of bases in the sequences of the Y and Z is not particularly limited and can be appropriately selected according to the purpose, preferably 2 to 12.

If the total number of bases in the sequences is less than 2, the regions may not contribute enough to the stabilization of the cleavage active region and improvement of cleavage efficiency. If the total number of bases in the sequences exceeds 12, in the case where the nucleic acid probe has a structure capable of forming the cleavage active region even before hybridization with the target nucleic acid, free nucleic acid probe, which has not hybridized with the target nucleic acid, forms the cleavage active region easily, cross hybridization of the nucleic acid probes to each other may occur, and detection accuracy, sensitivity, etc. may be reduced.

In the base sequence of the "Z" portion, 1 or several bases, which do not hybridize, may be present in a portion thereof (deletion, substitution, etc. may be present in a portion thereof).

Further, by the base sequence of the "Z" portion, normally in the nucleic acid probe, in the side opposite to the cleavage portion, a loop (loop portion) is formed.

The number of bases in the sequence of the loop portion is not particularly limited and can be appropriately selected according to the purpose. The reaction rate of the nucleic acid probe normally depends on the loop portion, and thus, the loop portion is preferably shorter due to its stability.

If the number of bases in the sequence of the loop portion is less than 2, a stem loop structure part described later becomes unstable due to disadvantage in terms of entropy, and efficiency of forming the cleavage active region may decrease.

When the cleavage portion is cleaved by the cleavage active region, the nucleic acid probe is divided into the first and second probe fragments.

The size of the first and second probe fragments is not particularly limited and can be appropriately selected according to the purpose. The size may be almost the same to each other or may be different to each other, and the size, which allows these to dissociate from the target nucleic acid easily, is preferable. This case is advantageous in that the signal (e.g. emission) produced as a result of the cleavage of the cleavage portion by the cleavage active region can be generated in a short time, enabling quick detection.

When the cleavage active region has DNAzyme activity, RNAzyme activity, ribozyme activity, etc. toward the cleavage portion, the cleavage active region normally has an active center site. When the metal ion exists in the active center site, the DNAzyme activity, RNAzyme activity, ribozyme activity, etc. can be generated.

The metal ion is not particularly limited and can be appropriately selected according to the purpose. Examples thereof include a magnesium ion, cobalt ion, and the like. Among these, the magnesium ion is suitable.

Methods for allowing the metal ion to exist in the active center site include, for example, a method in which the nucleic acid probe is placed in a solution containing the metal ion. In this case, the concentration of the metal ion in the solution is not particularly limited and can be appropriately selected according to the purpose. For example, 10 mM to 50 mM is preferable.

The cleavage active region can be designed in any method without limitation and the method can be appropriately selected according to the purpose. For example, when the cleavage active region is formed by a nucleic acid chain, e.g. DNA, the SELEX method mentioned above is suitable, for example.

In the SELEX method, a plurality of nucleic acids is brought into contact with a target (here, the cleavage portion), nucleic acids having a strong binding strength are selected and separated from the plurality of nucleic acids, amplified, further brought into contact with the target (the cleavage portion) together with a plurality of nucleic acids. By repeat of this, nucleic acids with high affinity to the target (the cleavage portion) are selected.

### - Cleavage portion -

The cleavage portion is enough if it can be cleaved specifically (can be cleaved by the cleavage active region) when the nucleic acid probe has hybridized to the target nucleic acid. The shape, conformation, size, material, etc. thereof are not particularly limited and can be appropriately selected according to the purpose.

The cleavage portion can be appropriately selected according to the type of the cleavage active region. For example, when the cleavage active region has the DNAzyme activity, preferably, the cleavage portion is DNA, when the cleavage active region has the RNAzyme activity, preferably, the cleavage portion is RNA, and when the cleavage active region has RNA cleavage activity, preferably, the cleavage portion is ribose.

The cleavage portion is cleaved by the cleavage active region. When the nucleic acid probe is designed so that the cleavage active region is formed when the nucleic acid probe has hybridized to the target nucleic acid, the cleavage portion is cleaved only when the nucleic acid probe has hybridized to the target nucleic acid. Thus, it is advantageous in that a false detection, etc. of the target nucleic acid can be prevented.

### - Locked structure -

It is preferable that the nucleic acid probe of the invention has formed a locked structure as a result of hybridization of portions to each other intramolecularly until the nucleic acid probe hybridizes to the target nucleic acid.

In this case, while the nucleic acid probe has the locked structure, i.e., until the nucleic acid probe hybridizes to the target nucleic acid, the cleavage active region is not formed. When the nucleic acid probe loses the intramolecular hybridization structure, i.e., only when the nucleic acid probe has hybridized to the target nucleic acid, the cleavage active region is formed. Thus, hybridization of the nucleic acid probe to the target nucleic acid and conformational change as typified by, e.g. cleavage of the cleavage portion can be completely linked, and it is advantageous in that a false detection, etc. can be prevented efficiently.

The locked structure is appropriately selected according to the purpose. Examples thereof include a stem loop structure, hairpin structure, and the like. These may be formed within the nucleic acid probe individually, or two or more thereof may be formed within the nucleic acid probe in combination. Among these, a stem loop structure is preferable, the stem loop structure means a three-dimensional hairpin structure, which nucleic acid generally forms, and normally comprises: a stem portion having a stem-like shape through a complementary binding; and a loop portion having a loop-like shape.

Preferably, the length of the base sequence of one strand of the complementary strands forming the hybridization (portions hybridized to each other, for example, the stem portion) in the locked structure is 4 nt to 8 nt.

If the length of the base sequence is less than 4 nt, the binding strength of the complementary strand in the hybridization region (e.g. the stem portion) is weak, which may not be sufficient and may cause a false detection, etc. In contrast, if the length of the base sequence exceeds 8 nt, the binding strength of the complementary strand in the hybridization region (e.g. the stem portion) is too strong, resulting in the failure of hybridization of the nucleic acid probe to the target nucleic acid or leading to the possible difficulty in hybridization.

### - Signal-generating portion -

The signal-generating portion in the nucleic acid probe of the invention is not particularly limited as long as it can generate a signal when the nucleic acid probe has hybridized to the target nucleic acid, and can be appropriately selected according to the purpose. Suitable examples of the signal-generating portion include: a light-emitting portion capable of emitting typically by hybridization of the nucleic acid probe to the target nucleic acid; a combination of a light-emitting portion generating emission and a quenching portion which quenches emission of the light-emitting portion when located adjacent to the light-emitting portion; or the like. The signal-generating portion can be used not only for detecting the presence or absence of the generation of signal but also as a dynamic parameter by which the behavior of the target nucleic acid in cells is monitored.

These may be provided in the nucleic acid probe individually, or two or more thereof may be provided in combination. Among these, a combination of the light-emitting portion and the quenching portion, or the like is suitable.

The signal is not particularly limited and can be appropriately selected according to the purpose. Examples thereof include emission, quenching, protein expression, emission of radiation, temperature change (e.g. heat generation), change in magnetic force (e.g. generation of magnetism), generation of a cleaved fragment, substance production, substance consumption, deformation, viscosity change, color change, UV absorption, pH change, optical rotation, isomerization, and the like. These may be used alone, or two or more may be used in combination.

The method or means for detecting the signal is not particularly limited and can be appropriately selected according to the purpose. For example, when the signal is emission, quenching, etc., it is detected by a photodetector, camera, etc. In the case of emission of radiation, it is detected by a sensitive film, etc. In the case of temperature change, by a thermocouple, temperature sensor, etc. In the case of change in magnetic force, by a magnetometer. In the case of generation of a cleaved fragment, by an electrophoresis, SDS-PAGE, western blotting, etc. In the case of substance production, by an antibody, HPLC, affinity chromatography, etc. In the case of substance consumption, by an IR spectrum, MS spectrum, etc. In the case of substance consumption, by an IR spectrum, MS spectrum, etc. In the case of deformation, by an electron microscope, etc. In the case of viscosity change, by a viscosity sensor, etc.

The combination of the light-emitting portion and the quenching portion is not particularly limited and can be appropriately selected according to the purpose. For example, those known as the technique of fluorescence resource energy transfer (FRET), etc. can be suitably adopted.

The light-emitting portion is not particularly limited as long as it can generate emission, and can be appropriately selected according to the purpose. Examples thereof include those containing a fluorescent substance, chemiluminescent substance, electrochemiluminescent substance, etc. or those formed of these substances.

These may be used alone or two or more may be used in combination. Among these, in the case where the quenching portion is present adjacent to the light-emitting portion, those of which emission is quenched by the action of the quenching portion are preferable, the fluorescent substance is more preferable in that visibility thereof is excellent and detection is easy.

The fluorescent substance is not particularly limited and can be appropriately selected according to the purpose. Examples thereof include anthracene, fluorescein, fluorescein isothyocianate (FITC), rhodamines such as tetramethyl rhodamine and sulforhodamine, dansyl chloride, Texas Red, AL 350, indocarbocyanine (CY), and the like.

The nucleic acid probe having the light-emitting portion is advantageous in that detection can be carried out, for example, with eyes.

The quenching portion is not particularly limited if it can quench the emission of the light-emitting portion when the quenching portion is located adjacent to the light-emitting portion, and the quenching portion can be appropriately selected according to the type, etc. of the light-emitting portion. Examples thereof include those containing a quenching substance, or those formed of the quenching substance.

The quenching substance is not particularly limited and can be appropriately selected according to the purpose. When the light-emitting portion is formed of the fluorescent substance, examples of the quenching substance include substances capable of absorbing the energy released when the fluorescent substance emits light, and the like. Suitable examples include substances allowing fluorescence resource energy transfer (FRET) between the light-emitting substance. Specific examples include tetramethylrhodamine isothiocyanate (TRTTC), dimethylaminobenzenesulfonyl (DABSYL), gold nanoparticles, Black Hole Quencher, and the like.

The nucleic acid probe having the quenching portion can quench emission of the light-emitting portion before the nucleic acid probe hybridizes to the target nucleic acid, i.e., when the quenching portion is present adjacent to the light-emitting portion. In contrast, after the nucleic acid probe has hybridized to the target nucleic acid, the cleavage portion is cleaved, divided into the first and second probe fragments, and these dissociate from the target nucleic acid. As a result, the quenching portion exists away from the light-emitting portion, resulting in the loss of the action of the quenching portion and allowing the light-emitting portion to generate emission. Therefore, the nucleic acid probe having the quenching portion is advantageous in that the presence of the target nucleic acid can be detected easily through the emission of the light-emitting portion, for example, with eyes.

The position at which the quenching portion is present in the nucleic acid probe is not particularly limited and can be appropriately selected according to the purpose. Preferably, the quenching portion is located adjacent to the light-emitting portion before the nucleic acid probe hybridizes to the target nucleic acid, and when the nucleic acid probe has hybridized to the target nucleic acid, i.e., after the nucleic acid probe has divided into the first and second probe fragments as a result of the cleavage of the cleavage portion, the quenching portion is preferably located apart from the light-emitting portion. Specifically, it is more preferable that the quenching portion is present in the second probe fragment when the light-emitting portion is present in the first probe fragment. Conversely, when the light-emitting portion is present in the second probe fragment, it is more preferable that the quenching portion is present in the first probe fragment, in other words, the quenching portion and the light-emitting portion are located via the cleavage portion. This case is advantageous in that upon cleavage of the cleavage portion, it is possible for the quenching portion to be present in other probe fragment different from that of the light-emitting portion.

Until the cleavage portion is cleaved, the quenching portion can be located at any distance from the light-emitting portion within a range where the quenching effect of the quenching portion occurs, and the distance can be appropriately selected according to the purpose. For example, the number of base present between the terminal base of the quenching portion on the light-emitting portion side and terminal base of the light-emitting portion on the quenching portion side is preferably 5 nt to 30 nt.

If the number of base is less than 5, molecular design, synthesis, etc. may not be easy because the distance between the quenching portion and light-emitting portion is too close. If the number of base exceeds 30, quenching effect of the quenching portion may not be enough.

Specific combination of the light-emitting substance and eliminating substance is not particularly limited and can be appropriately selected according to the purpose. Suitable examples include a combination of fluorescein isothyocianate (FITC) and tetramethylrhodamine isothiocyanate (TRITC), a combination of dimethylaminobenzenesulfonyl (DABSYL) and fluorescein, and the like.

In the above description, explanation was mainly given to the case where the nucleic acid probe is designed so that when the nucleic acid probe has hybridized to the target nucleic acid, emission of the light-emitting portion is generated. However, the invention is not limited to this case, the nucleic acid probe may be designed so that the emission of the light-emitting portion is generated before the nucleic acid probe hybridizes to the target nucleic acid, and when the nucleic acid probe has hybridized to the target nucleic acid, emission of the light-emitting portion is quenched. The nucleic acid probe of the invention may be designed in a form not having the labeling substance (e.g. the light-emitting portion and the quenching portion). The detection of the hybridization to the target nucleic acid by the nucleic acid probe can be carried out by detecting e.g. a probe fragment, which is generated by the cleavage as a result of the hybridization, typically by electrophoresis.

In the example of FIG. 3, the fluorescent substance F and quenching substance Q each are preferably provided at the position via the cleavage site 12 by the self-cleaving nucleic acid enzyme. The principle is based on fluorescence resource energy transfer (FRET). In order for FRET to occur, the distance between substances, a fluorescent substance (F) and quenching substance (Q), is particularly important. In the state where the nucleic acid probe 2 of the invention has hybridized to the target nucleic acid 1, the distance between substances, F and Q, is designed to such a distance that FRET occurs and emission is quenched. Thereafter, self-cleaving nucleic acid enzyme works, oligonucleotide is cleaved at a predetermined cleavage site, and then each of the cleaved fragments dissociates since it cannot keep the binding with the target nucleic acid. As a result, the fluorescent substance released from FRET emits fluorescence. In this way, a target nucleic acid can be detected or determined quantitatively from the increase of intensity of fluorescence.

Further, such FRET method is preferable because it enables detection of a target nucleic acid of interest without separating a hybridized nucleic acid probe and a nucleic acid probe which does not hybridize, and process is simplified.

The fluorescent substance and the quenching substance are preferably apart at a distance of about 5 bases to about 30 bases via cleavage site in a state where they have hybridized to the target nucleic acid, but are not always limited to this case.

As the labeling substance to be introduced into a nucleic acid probe, a combination consisting of an energy-donating substance and energy-accepting substance (FITC/TRITC) may be adopted.

The nucleic acid probe of the invention can be used in a form fixed on the surface of a support such as a glass substrate, so called as "DNA chip", and can also be used as Lab-on-Chip.

### - Other part -

The other part is not particularly limited and can be appropriately selected as long as it does not impair the effect of the invention. Examples thereof include various kinds of markers, water-soluble linkers, and the like.

The various kinds of markers are not particularly limited and can be appropriately selected according to the purpose. Examples thereof include radioactive labels, quantum dot labels, protein labels, and the like.

When the nucleic acid probe is used immobilized on a support, it is preferable that the water-soluble linker be introduced between the nucleic acid probe and the support.

The support is not particularly limited and can be appropriately selected according to the purpose. Examples thereof include resin particles, plates, bottom or side of containers, and the like. The detection of the target nucleic acid can be carried out easily by fixing the nucleic acid probe to e.g. a detection kit beforehand.

The water-soluble linker is not particularly limited and can be appropriately selected according to the purpose. For example, an ethylene glycol linker can be used. The length of the water-soluble linker can be determined to an appropriate length according to the structure, type, etc. of support.

When the nucleic acid probe is used by introducing into cells, it is preferable that part of or all nucleic acids in the nucleic acid probe be subjected to 2'-OMe treatment (treatment in which a hydroxyl group at the 2' position of sugar is substituted with a methoxy group). This allows for the inhibition of non-specific cleavage of the nucleic acid probe by an intracellular enzyme, improving detection accuracy of the target nucleic acid.

### - Usage and utility -

The nucleic acid probe of the invention can detect even very small amounts of the target nucleic acid of, for example, viruses, bacteria, animal cells, plant cells, is suitably applicable to a gene therapy, test for the presence of food-poisoning bacteria, diagnosis of tooth decay or periodontal disease, blood test, etc., and is suitable for the nucleic acid chip, method for detecting a target nucleic acid, method for screening a drug, apparatus for detecting a gene, and gene diagnosis method, of the invention described later.

The nucleic acid probe of the invention can be suitably applied to or is suitably applicable to, for example, a blood test (prevention of viral infection during blood transfusion), hygiene maintenance of blood products such as a whole blood, albumin product, and blood-clotting product, virus screening during artificial insemination, etc., test for venereal disease, analysis of the condition of a patient with nervous disease. More specifically, since a trace amount of virus or bacteria are contained in saliva, for example, as a HIV infection test and diagnosis of tooth decay or periodontal disease, only addition of the nucleic acid probe of the invention in the saliva sample allows positive or negative test to be carried out conveniently and easily, for example, with eyes.

The nucleic acid probe of the invention is also applicable suitably in the regions such as a crime investigation, forensic medicine, and archaeology.

When the nucleic acid probe of the invention is used for detecting the target nucleic acid, the nucleic acid probe may be used alone, or may be used in the presence of helper oligonucleotide (together with helper oligonucleotide).

The helper oligonucleotide is not particularly limited as long as it has a function of helping or facilitating the nucleic acid probe to hybridize to the target nucleic acid, and can be appropriately selected from those known in the art according to the purpose, but preferably, those having a function of helping the stem loop structure change into a straight-chain structure. When the nucleic acid probe has the locked structure, the helper oligonucleotide can be used particularly suitably in combination with the nucleic acid probe.

The length of the helper oligonucleotide, i.e., the number of bases in the sequence, is not particularly limited and can be appropriately selected according to the purpose, for example, preferably 15 to 20.

### - Target nucleic acid -

Here, the target nucleic acid, which is the subject to be detected by the nucleic acid probe of the invention, is not particularly limited and can be appropriately selected according to the purpose. The target nucleic acid may be any of purified and unpurified one and may be any of nucleic acid such as, RNA, DNA, PNA, and nucleic acid modified artificially. The base sequence of the target nucleic acid can be determined by any method without limitation, and the method can be appropriately selected according to the purpose. Examples thereof include Maxam-Gilbert method, dideoxy method, and the like.

The sample, in which the target nucleic acid is present, is not particularly limited and can be appropriately selected according to the purpose. The sample may be, for example, collected samples or prepared samples such as a blood, serum, blood plasma, fecal matter, urine, expectoration, bone marrow fluid, sweat, tear, saliva, semen, water e.g. tap water, and pretreated samples thereof; or may be cells (including live cells), viruses or the like.

The cell is not particularly limited and can be appropriately selected according to the purpose. Suitable examples thereof include an animal cell, plant cell, fungus, yeast, and the like.

The animal cell is not particularly limited and can be appropriately selected according to the purpose. Examples thereof include embryonic stem cells such as a fertilized ovum, ES cell, EG cell, and EC cell, embryonal carcinoma cells, hematopoietic stem cells, cancer cell line, which have become cancerous and immortalized, cell line from each organ, red blood cells, lymphocytes, leucocytes, and the like. The animal cell also includes cells of transgenic animal transformed from developmental time.

The organism from which the animal cell is derived may be any of, for example, mammals, reptiles, birds, amphibian, fishes, and insects.

Examples of the mammals include carnivores, primates, herbivores, rodents, and the like. Specific examples include mice, rats, hamsters, cattle, horses, pigs, goats, wild boars, elephants, giraffes, pandas, dogs, cats, bears, rabbits, whales, dolphins, monkeys, humans, and the like.

Examples of the reptiles include Chelonia, Ophidia, Crocodilia, Rhynchocephalia, Pholidota, and the like. Specific examples include tortoises, sea turtles, red-eared turtles, Reeve's turtles, lizards, iguanas, chameleons, geckos, pythons, Colubridae, cobras, and the like.

Examples of the birds include a duck, goose, albatross, chicken, Japanese crested ibis, sea gull, ostrich, tinamou, sparrow, crow, pheasant, gfisher, hill myna, Okinawa rail, white stork, crane, little cuckoo, woodpecker, owl, curassow, hemipode, parrot, and the like.

Examples of the amphibian include a frog, newt, salamander, salamander, and the like.

Examples of the fishes include a freshwater fish, saltwater fish, and the like. Specific examples of the freshwater fish include a arowana, guppy, catfish, Japanese bitterling, carp, killifish, yamame (a kind of trout), charr, piranha, lungfish, cichlid, catfish, and the like. Specific examples of the saltwater fish include a ray, shark, anemone fish, sillaginoid fish, goby, tuna, flatfish, ocean sunfish, manta, anemone fish, angelfish, discus, and the like.

Examples of the insects include a beetle, stag beetle, dragonfly, wasp, cricket, bell cricket, longicorn; ant, cockroach, mantis, cicada, giant water bug, fly, grasshopper, firefly, butterfly, and the like.

The plant cell is not particularly limited, can be appropriately selected according to the purpose, including, for example, cells derived from parts such as a flower, stem, root, and leaf, and may be protoplasts thereof. The type of the plant is not particularly limited and can be appropriately selected according to the purpose. The plant cell includes, for example, cells derived from a seed plant, ferns, bryophytes, algae, and also includes a transgenic plant into which the polynucleotide is introduced at the time of development.

Examples of the seed plant include a gymnosperm, angiosperm, and the like. Specific examples of the seed plant include a rice plant, wheat, barley, Japanese cherry, dandelion, pine tree, tulip, sunflower, cryptomeria, beech, eggplant, lotus, oilseed rape, pink, broad bean, camellia, Japanese parsley, maize, Welsh onion, hydrilla, taro, dayflower, ginkgo, cycad, Japanese cypress, Japanese nutmeg tree, Japanese larch, Chinese juniper, chrysanthemum, perilla, gentian, primrose, orchid, lily, iris, and the like.

Examples of the fern include Athyrium niponicum, a bracken, field horsetail, flowering fern, tree fern, quillwort, club moss, scouring rush, and the like.

Examples of the bryophytes include a hair-cap moss, sphagnum, Andreaeceae, luminous moss, Mniaceae, Atrichum undulatum, liverwort, Conocephalum conicum, scale moss, Riccia glauca L., Haplomitrium mnioides, Riccia fluitans, and the like.

Examples of the algae include a chlamydomonas, green laver, chlorella, pond scum, sea staghorn, sea lettuce, aegagropila, and the like.

The fungus is not particularly limited and can be appropriately selected according to the purpose. Examples thereof include mushrooms, true fungi, bacteria, and the like.

Examples the mushrooms include a shiitake mushroom, matsutake mushroom, Agaricus, king oyster, hen of the woods, monkeyhead mushroom, bunashimeji mushroom (Hypsizygus marmoreus), Pleurotus cornucopiae var.citrinopileatus, nameko mushroom, oyster mushroom, honashimeji mushroom (Lyophyllum shimeji), Flammulina velutipes, and the like.

The true fungi can be mainly classified into yeast and filamentous fungus.

Examples of the yeast include Candida albicans, C. glabrada, C. tropicalis, C. parapsilosis, C. parapsilosis, Cryptococcus neoformans, Saccharomyces cerevisiae, and the like. Examples of the filamentous fungus include Trichophytin rubrum, Microsporum canis, Alternaria alternate, Alternaria panax, Bipolaris brizae, and the like.

Examples of the bacteria include Gram-positive bacteria, Gram-negative bacteria, and the like. The bacteria may be any of anaerobic bacteria, facultative anaerobe, microaerophile, aerobic bacteria, etc. Specific species of the bacteria include, for example, Staphylococcus aureus, Streptococcus pyogenes, Enterococcus faecalis, Bucillus anthracis, Batcillus subtilis, Clostridium tetani, Listeria monocytogenes, Pseudomonas aeruginosa, Escherichia coli, Heamophilus influenzae, Neisseria gonorrhoeae, Mycobacterium tuberculosis, Corynebacterium glutamicum, Streptomyces antibioticus, Salmonella Typhi, Edwardsiella tarda, Citrobacter freundii, Vibrio parahaemolyticus, Morganella morganii, Seratia marcescens, Klebsiella pneumoniae, Shigella dysenteriae, Yersinia pestis, Treponema pallidum, Leptospira interrogans, Campylobactor jejuni, Lactobacillus latis, Actinobacillus actinomycetemcomitance, Purphyromonas gingivalis, Prevotella intermedia, Bacteroides forsythus, Treponema denticola, and further include Chlamydiaceae, Rickettsiaceae, Mycoplasmataceae, and the like.

Suitable examples of the bacteria also include periodontal-disease-causing bacteria, cariogenic bacteria, food-poisoning-causing bacteria, and the like.

Further, other microbes include a protest, plankton, and the like.

Examples of the protest include Entamoeba histolytica, Entamoeba coli, Trichomonas vaginalis, a trypanosome, malaria, Pneumocystis Pneumocystis carini, Cryptomonas, Euglena, filarial, whipworm, and the like.

Examples of the plankton include a water flea, cyclops, rotifer, and the like.

The virus is not particularly limited and can be appropriately selected according to the purpose. Examples thereof include a retrovirus, adenovirus, herpesvirus, Sendai virus, bacteriophage, and the like.

Examples of the retrovirus include a murine leukemia virus, human immunodeficiency virus, Human T-Lymphofiropic Virus, and the like.

Examples of the adenovirus include canine adenovirus type 1, canine adenovirus type 2, human adenovirus type 1, human adenovirus type 2, and the like.

Examples of the Sendai virus include those mainly derived from mouse, and it is known that the Sendai virus does not infect human.

Examples of the bacteriophage include a T4 phage, T5 phage, T7 phage, λ phage, M13, and the like.

Further, the sample includes a plasmid in E. coli, cosmid, and the like. Examples of the plasmid include pBR322, pBR325, pAT153, pUC18, pUC19, pSP-RLUC, and the like.

### (Nucleic acid chip)

The nucleic acid chip of the invention is not particularly limited except that it comprises a support and the nucleic acid probe of the invention immobilized thereon, and configurations known in the art can be appropriately adopted according to the purpose. Among them, a configuration is preferable in which the nucleic acid probe is immobilized on the support via the water-soluble linker.

Suitable examples of the subject on which the nucleic acid probe is immobilized include supports known in the art. The nucleic acid chip comprising the support and the nucleic acid probe immobilized thereon, can be used suitably as a DNA chip.

The support is not particularly limited, can be appropriately selected according to the purpose, and, for example may be formed of, an inorganic material or may be formed of, an organic material.

Examples of the inorganic material include an inorganic polymer, metal, ceramic, semiconductor, magnet, paramagnet, apatite, and the like.

Suitable examples of the inorganic polymer include a carbon, amorphous carbon obtained through carbonization by baking a thermosetting resin, graphite, and the like.

Suitable examples of the metal include gold, platinum, silver, copper, iron, aluminum, and the like.

Suitable examples of the ceramic include alumina, silica, silicon nitride, silicon carbide, glass, rock crystal, silica gel, and the like.

Suitable examples of the semiconductor include silicon.

These inorganic materials may be used alone, or two or more may be used in combination.

Examples of the organic material include a plastic, naturally-occurring polymer, and the like.

Examples of the plastic include polyethylene, polystyrene, polycarbonate, polypropylene, polyamide, phenolic resin, epoxy resin, polycarbodiimide resin, polyvinyl chloride, polyvinylidene fluoride, polyethylene fluoride, polyimide, acrylic resin, ceramic, and the like.

Examples of the naturally-occurring polymer include polyamino acid, cellulose, chitin, chitosan, alginic acid, derivatives thereof, and the like.

The shape of the support can be selected from the viewpoint that how the nucleic acid chip is to be designed because normally, the shape of the support determines that of the nucleic acid chip, and the shape is not particularly limited. Suitable examples of the shape include film-like, plate-like, particle-like (e.g. beads), a variety of molded articles, and the like. When the shape is particle-like, the nucleic acid chip can be designed for example, as a DNA microbeads array in which the nucleic acid probe immobilized on a fluid bead surface. Examples of the variety of molded articles include a strip, well or strip of multiwell plate, tube, mesh, continuously expanded foam, membrane, paper, needle, fiber, plate, hollow fiber, slide, cell vessel, and the like.

The size of the nucleic acid chip is not particularly limited, and can be appropriately selected according to the purpose.

The nucleic acid probe can be arranged at any place on the nucleic acid chip without limitation and arrangement can be appropriately selected according to the purpose. For example, the nucleic acid probe may be arranged on the entire surface of the support or on part of the surface thereof, or may be arrayed on the surface of the support.

The type of the nucleic acid probe to be arranged on the nucleic acid chip is not particularly limited, can be appropriately selected according to the purpose, and one type may be arranged alone or two or more types may be arranged. In the latter case; the arrangement of each nucleic acid probe in an array enables qualitative detection, quantitative detection, etc. of a variety of and/or a large amount of genes simultaneously, and such nucleic acid chip is suitably applicable to gene diagnosis, etc. At this time, to design so that color of emission is different by the type of the nucleic acid probe (to design as multicolor probe) is advantageous in that only by observing emission color, for example, with eyes, the presence or absence of a particular target nucleic acid can be judged instantly.

The nucleic acid chip of the invention can be suitably used for detection or analysis of the target nucleic acid, and can be suitably used for the method for detecting a target nucleic acid, method for screening a drug, gene diagnosis method, etc. of the invention described later.

The nucleic acid chip of the invention can detect even very small amounts of the target nucleic acid of, for example, viruses, bacteria, animal cells, plant cells, is suitably applicable to a gene therapy, etc., and is suitable for the method for detecting a target nucleic acid, method for screening a drug, apparatus for detecting a gene, and gene diagnosis method, of the invention described later.

The nucleic acid chip of the invention can be suitably applied to or is suitably applicable to; for example, a blood test (prevention of viral infection during blood transfusion), hygiene maintenance of blood products such as a whole blood, MAP, FFP, albumin product, and blood-clotting product, virus screening during artificial insemination, etc., test for venereal disease, analysis of the condition of a patient with nervous disease. More specifically, since a trace amount of virus or bacteria are contained in saliva, for example, as a HIV infection test, only use of nucleic acid chip of the invention in the saliva sample allows positive or negative test to be carried out conveniently and easily, for example, with eyes.

The nucleic acid chip of the invention is also applicable suitably in the regions such as a crime investigation, forensic medicine, and archaeology.

### (Method for detecting target nucleic acid)

The target detection method of the invention includes the following first to third target detection methods.

The first method for detecting a target nucleic acid is a method for detecting a nucleic acid in which a target nucleic acid is detected using a nucleic acid probe, the nucleic acid probe is designed such that it undergoes a conformational change after hybridized to the target nucleic acid, the nucleic acid probe, which underwent the conformational change, have decreased binding strength of hybridization, and dissociates from the target nucleic acid.

The second method for detecting a target nucleic acid is a method for detecting a target nucleic acid using a nucleic acid probe comprising: a complementary region having a sequence complementary to at least a portion of the base sequence of a target nucleic acid; and a nucleic acid enzyme forming region capable of forming a self-cleaving nucleic acid enzyme. The second method for detecting a target nucleic acid comprises: a hybridization step in which the nucleic acid probe is allowed to bind to a target nucleic acid complementarily; a dissociation step in which the self-cleaving nucleic acid enzyme is formed after the formation of hybridization, the nucleic acid probe undergoes a conformational change, as a result, the binding strength of hybridization decreases, and the nucleic acid probe dissociates from the target nucleic acid; and a detection step in which at least a part of the nucleic acid probe dissociated in the dissociation step is detected.

The third method for detecting a target nucleic acid comprises: a hybridization step in which the nucleic acid probe of the invention is allowed to hybridize to a target nucleic acid; and a target nucleic acid detection step in which the presence of the target nucleic acid is detected by detecting a signal generated from the nucleic acid probe upon hybridization to the target nucleic acid, and may further comprise other steps appropriately selected according to necessity.

In the method for detecting a target of the invention, basically, only operation that include a nucleic acid probe is allowed to hybridize to a target nucleic acid present in a sample is required, after this operation, spontaneous conformational change of nucleic acid probe, decrease of binding strength of hybridization, dissociation of nucleic acid probe, and accumulation of signal take place, enabling the measurement of a target nucleic acid. In this way, operation is quite easy, thus a complex or special apparatus and a special reagent, etc. are not required, and gene diagnosis can be carried out quickly at required places, when required, for example; at a clinic, home, school, and workplace.

When the detection of a target nucleic acid is carried out in cells, the nucleic acid probe is required to be introduced into cells. The nucleic acid probe can be introduced into cells by any method without limitation, and the method can be appropriately selected according to the type, etc. Examples thereof include known gene transfer methods such as a calcium phosphate method, liposome method, electroporation method, and sonoporation method.

In the method for detecting a nucleic acid of the invention, a nucleic acid probe is preferably used which comprises a labeling substance that emits different signals before and after a nucleic acid probe dissociates from the target nucleic acid.

Such configuration makes it easier to view a phenomenon such as hybridization of the nucleic acid probe to the target nucleic acid or dissociation as a change of signal, making the detection of target nucleic acid easier.

The method for detecting a target nucleic acid of the invention is a method for detecting a target nucleic acid using a nucleic acid probe comprising: a complementary region having a sequence complementary to at least a portion of the base sequence of a target nucleic acid; and a nucleic acid enzyme forming region capable of forming a self-cleaving nucleic acid enzyme. The method for detecting a target nucleic acid comprises: a hybridization-forming step in which the nucleic acid probe is allowed to bind to a target nucleic acid complementarily; a dissociation step in which the self-cleaving nucleic acid enzyme is formed after the formation of hybridization, the nucleic acid probe undergoes a conformational change, as a result, the binding strength of hybridization decreases, and the nucleic acid probe dissociates from the target nucleic acid; and a detection step in which at least a part of the nucleic acid probe dissociated in the dissociation step is detected.

By such method, the labeling substance on the nucleic acid probe, which hybridized with a target nucleic acid once, can be detected positively. Thus, the method for detecting a nucleic acid is excellent in operability and enables the detection of a target nucleic acid with ease and high accuracy.

Further, since only complementary region of a nucleic acid probe is allowed to hybridize with a target nucleic acid, the time needed to accomplish hybridization can be reduced, enabling a test to be done rapidly.

According to the method for detecting a target nucleic acid of the invention, sample DNA (RNA) is not required to be amplified by e.g. PCR because the nucleic acid probe itself not only recognizes a target nucleic acid but also amplifies signal, restriction enzyme, etc., as required before, are not required, restriction enzyme site is not required in the nucleic acid probe, and further, addition of chemical substances, etc. is not required. Reaction proceeds at a constant temperature. Therefore, the method can be used as a detection method of a target nucleic acid in cells.

### - Hybridization step -

The hybridization step is a step in which the nucleic acid probe of the invention is allowed to hybridize to the target nucleic acid described above.

The condition, etc. of the hybridization are not particularly limited and can be appropriately selected according to the purpose. Preferably, the hybridization is carried out under so-called "stringent" conditions. pH is preferably 7.0 to 8.5, temperature is preferably 30°C to 60°C, and hybridization is preferably carried out in a solution with salt concentrations from 0.01 M to less than 0.1 M sodium ion.

In the hybridization step, the helper oligonucleotide mentioned above may be used in combination.

The helper oligonucleotide is not particularly limited as long as it has a function of helping or facilitating the nucleic acid probe to hybridize to the target nucleic acid, and can be appropriately selected from those known in the art according to the purpose, but preferably, those having a function of helping the locked structure (stem loop structure) change into a straight-chain structure.

When the nucleic acid probe has the locked structure, the helper oligonucleotide can be used particularly suitably in combination with the nucleic acid probe.

### - Target nucleic acid detection step -

The target nucleic acid detection step is a step in which the target nucleic acid is detected by detecting the signal generated when the nucleic acid probe of the invention has hybridized to the target nucleic acid.

The signal include, as mentioned above, emission, quenching, emission of radiation, temperature change (e.g. heat generation), change in magnetic force (e.g. generation of magnetism), generation of a cleaved fragment, substance production, substance consumption, deformation, viscosity change, for example. These may be used alone, or two or more may be used in combination.

The method or means for detecting the signal is not particularly limited and can be appropriately selected according to the purpose. For example, when the signal is emission, quenching, etc., it is detected by a photodetector, camera, etc. In the case of emission of radiation, it is detected by a sensitive film, etc. In the case of temperature change, by a thermocouple, temperature sensor, etc. In the case of change in magnetic force, by a magnetometer. In the case of generation of a cleaved fragment, by an electrophoresis, SDS-PAGE, western blotting, etc. In the case of substance production, by an antibody, HPLC, affinity chromatography, etc. In the case of substance consumption, by an IR spectrum, MS spectrum, etc. In the case of substance consumption, by an IR spectrum, MS spectrum, etc. In the case of deformation, by an electron microscope, etc. In the case of viscosity change, by a viscosity sensor, etc.

First and second probe fragments are generated by the cleavage of the nucleic acid probe. When the signal is emission of the light-emitting portion which is present in the first probe fragment, in the target nucleic acid detection step, the presence of the target nucleic acid is detected by detecting the emission. At this time, the emission of the light-emitting portion can be detected by any method without limitation, and the method can be appropriately selected according to the purpose. When the light-emitting portion is composed of the light-emitting substance and the light-emitting substance is a fluorescent substance; the emission (fluorescence) can be detected with, for example, eyes or a fluorescence microscope easily. When the light-emitting substance is the chemiluminescent substance, the emission can be detected easily using a sensitive film, etc. Further, in the invention, the emission may be detected using a CCD camera. This case is advantageous in that digital analysis can be carried out, treatment or processing of data is easy, and qualitative and/or quantitative analysis of the target nucleic acid can be carried out highly accurately.

The method for detecting a target nucleic acid of the invention can detect even very small amounts of the target nucleic acid of, for example, viruses, bacteria, animal cells, plant cells, is suitably applicable to a gene therapy, test for the presence of food-poisoning bacteria, diagnosis of tooth decay or periodontal disease, blood test, etc.

The method for detecting a target nucleic acid of the invention can be suitably applied to or is suitably applicable to, for example, a blood test (prevention of viral infection during blood transfusion), hygiene maintenance of blood products such as a whole blood, MAP; FFP, albumin product, and blood-clotting product, virus screening during artificial insemination, etc., test for venereal disease, analysis of the condition of a patient with nervous disease. Further, the method for detecting a target nucleic acid of the invention is also applicable suitably in the regions such as a crime investigation, forensic medicine, and archaeology.

Here, one example of the method for detecting a target nucleic acid of the invention will be described with reference to drawings.

Initially, a nucleic acid probe 2 of the invention is allowed to hybridize to a target nucleic acid 1 ("A" in FIG. 1). In a hybridization-forming step, reaction condition can be appropriately set, and hybridization is carried out by methods known in the art, and is carried out under so-called "stringent" conditions. For example, hybridization is carried out using a solution with pH 7.0 to 8.5, at the temperature of 30°C to 60°C, and salt concentrations from 0.01 M to less than 0.1 M magnesium ion. It is preferable that normally, the target nucleic acid is denatured to a single strand beforehand by e.g. high-temperature treatment.

Next, the hybridized nucleic acid probe forms a self-cleaving nucleic acid enzyme 7 under given conditions ("A" in FIG. 1). This self-cleaving nucleic acid enzyme 7 recognizes a specific sequence and exhibits enzyme activity in the target nucleic acid 1. When the self-cleaving nucleic acid enzyme 7 has cleavage activity; nucleotides are cleaved at the cleavage portion 12 having a specific sequence ("B" in FIG. 1). The nucleic acid probe 2, cleaved in this way, cannot keep a binding state with the target nucleic acid 1 and dissociates ("C" in FIG. 1). This results in that the fluorescent substance F on a nucleic acid probe fragment is free from the effect of the quenching substance Q, emitting a fluorescence signal. This signal is detected or analyzed in the following detection step.

Specifically, the nucleic acid probe 2 comprises complementary base sequences 3a and 3b, which can hybridize to the target nucleic acid 1, at both ends thereof (See FIGS. 2 and 3). In the hybridization step, as shown in FIG. 1, when base sequences 3a and 3b hybridize to the nucleic acid sequence 1 in the presence of the target nucleic acid 1 ("A" in FIG. 1), nucleic acid enzyme forming region 6 adopts a three dimensional conformation, and thus the cleavage active region 7 is formed, yielding cleavage activity (ribozyme activity) toward the cleavage portion 12. The cleavage portion 12 (ribose moiety) is cleaved (self-cleaved), divided into the first probe fragment comprising the light-emitting substance F and the second probe fragment comprising the quenching substance Q ("B" in FIG. 1), and each of the probe fragments is dissociated from the target nucleic acid 1. As a result, the light-emitting substance F and quenching substance Q, which had located adjacent to each other, are located apart from each other ("C" in FIG. 1). When the light-emitting substance F and quenching substance Q are located adjacent to each other, emission of the light-emitting substance F is in a quenched state 8 by the action of the quenching substance Q through fluorescence resource energy transfer (FRET) between them; however, the fluorescence resource energy transfer is lost, and quenching effect of the quenching substance Q does not occur any more, resulting in that the light-emitting substance F emits light ("C'' in FIG. 1). This emission is generated every time the nucleic acid probe hybridizes to the target nucleic acid 1, and is amplified through the repeat of this reaction (catalytic process; cycle of A to C in FIG. 1). Namely, when the nucleic acid probe 2 is cleaved, the first and second probe fragments cannot keep hybridization and dissociates from the target nucleic acid 1. Then, the target nucleic acid 1 becomes free, and thus a new nucleic acid probe 2 hybridizes, leading to the repeat of the cycle of A to C in FIG.1. The nucleic acid probe 2 can make the target nucleic acid act as an allosteric effector. Therefore, even if the target nucleic acid 1 is present in very small amounts (< 10 ng/µl) or has a short life-span, the presence thereof can be detected easily, for example, with eyes by amplifying the light emitted by the nucleic acid probe.

In the target nucleic acid detection step, emission by the light-emitting substance F is detected. This detection is carried out by appropriately selecting a method depending on the character of the nucleic acid probe and on a labeling substance. For example, when the nucleic acid probe is labeled with a fluorescent substance, the detection is carried out by a fluorescence microscope, etc. When labeled with a radioisotope, the detection is carried out by an autoradiography, etc. When labeled with a chemiluminescent substance, the detection is carried out by analysis in which a sensitive film is used, or by digital analysis in which a CCD camera is used. As a result, qualitative and/or quantitative analysis of the target nucleic acid can be carried out.

When a nucleic acid probe which does not comprise a labeling substance is used, a target nucleic acid can be detected; for example, by confirming cleaved nucleic acid probe fragments by gel electrophoresis, etc.

In the method for detecting a nucleic acid of the invention, preferably, the nucleic acid probe is cleaved in a nucleic acid enzyme reaction step.

As described above, the activity of the self-cleaving nucleic acid enzyme is not particularly limited, but a nucleic acid probe is preferably cleaved through exhibition of cleavage activity. Each fragment, generated by the cleavage of a nucleic acid probe, cannot keep hybridization with the target nucleic acid and dissociates from the target nucleic acid. Thereafter, a new nucleic acid probe binds to the target nucleic acid. As a result, a reaction cycle, which enables such signal amplification, can be formed easily.

Further, in the method for detecting a nucleic acid of the invention, preferably, the nucleic acid probe is immobilized on a support. This makes it possible to configure devices for analyzing a nucleic acid such as a DNA chip and DNA microarray, enabling a large amount of gene date to be obtained highly sensitively.

Furthermore, according to the device for analyzing a nucleic acid in which the nucleic acid probe of the invention is used, time course of mRNA abundance of each gene can be observed, for example, by removing mRNA from a cell or organ at different times after administration of drug and by measuring it with e.g. a DNA chip on which the nucleic acid probe of the invention is immobilized. The increase of mRNA abundance means that genetic information from a DNA molecule is transcribed actively, corresponding to the increased activity of a gene. By the computer analysis of the time change of this activity of the gene, information on gene pathway, etc. can be obtained.

Additionally, by constructing a device for analyzing a nucleic acid in which two or more nucleic acid probes with different base sequences are immobilized on the same support, a variety of target nucleic acids can be detected or measured at a time.

Further, other example of the method for detecting a target nucleic acid of the invention will be described with reference to drawings.

As shown in left figure in FIG. 5A and "B" in FIG. 6, in the hybridization step, when the target nucleic acid (target) is present, the nucleic acid probe comprises two base sequences which are complementary to the target nucleic acid and are capable of hybridizing to the target nucleic acid at both ends (See FIG. 5A and B in FIG. 6). Thus, when the two base sequences hybridize to the nucleic acid (FIG. 5A and B in FIG. 6), the cleavage active region is formed, yielding cleavage activity (ribozyme activity) toward the cleavage portion (In the C in FIG. 6, magnesium ion is positioned in the center of the cleavage active region, thereby resulting in a state where cleavage activity is exhibited or improved). The cleavage portion (ribose portion) is cleaved (self-cleaved), divided into a first probe fragment comprising the light-emitting substance F and a second probe fragment comprising the quenching substance Q (D in FIG. 6), and each of the probe fragments is dissociated from the target nucleic acid. The light-emitting substance F and quenching substance Q, which had located adjacent to each other, are located apart from each other when the nucleic acid probe has hybridized to the target nucleic acid (B in FIG. 6), and thus quenching effect of the quenching substance Q does not occur, resulting in that the light-emitting substance F emits light. Note that the light-emitting substance F emits light almost at the same time as the cleavage portion is cleaved as a result of hybridization of the nucleic acid probe to the target nucleic acid. The emission by the light-emitting substance F occurs every time the nucleic acid probe hybridizes to the target nucleic acid, and is amplified through the repeat of this reaction (cycle of A to D in FIG. 6). Namely, when the nucleic acid probe is cleaved, the first and second probe fragments cannot keep hybridization and dissociates from the target nucleic acid. Then, the target nucleic acid becomes free, and thus a new nucleic acid probe hybridizes, leading to the repeat of the cycle of A to D in FIG. 6. The nucleic acid probe can make the target nucleic acid an allosteric effector. Therefore, even if the target nucleic acid is present in very small amounts (< 10 ng/µl) or has a short life-span, the presence thereof can be detected easily, for example, with eyes by amplifying the light emitted by the nucleic acid probe.

In the target nucleic acid detection step, emission by the light-emitting substance F is detected. This detection may be carried out with eyes or a fluorescence microscope, CCD camera, etc. may be used.

The method for detecting a target nucleic acid of the invention is suitable for detecting various kinds of target nucleic acids in trace amounts highly sensitively, highly accurately, and highly quickly and is also suitably applicable to the method for screening a drug of the invention described later.

### (Method for screening drug)

The method for screening a drug of the invention comprises a hybridization step and target nucleic acid detection step, and may further comprise other steps appropriately selected according to necessity.

The hybridization step is a step in which the nucleic acid probe of the invention is allowed to hybridize to the target nucleic acid, which is expressed or is not expressed due to administration of a drug, in the same way as described above.

### - Target nucleic acid detection step -

The target nucleic acid detection step is a step in which the signal is detected which is generated when the nucleic acid probe of the invention has hybridized to the target nucleic acid to thereby detect the target nucleic acid. The target nucleic acid detection step is the same as the target nucleic acid detection step in the method for detecting a target nucleic acid.

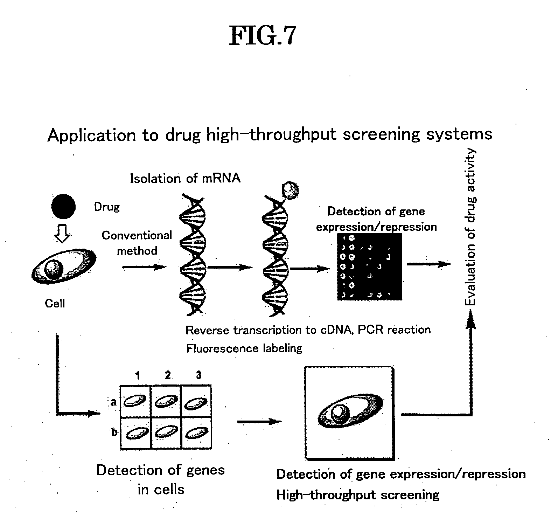

In the method for screening a drug of the invention, a drug is screened by the presence or absence of the target nucleic acid which is detected in the target nucleic acid detection step. Specifically, the effect or side effect of the drug can be screened efficiently by detecting the amplified emission, and observing the change of expression level of a gene after the administration of a drug. In addition, this screening can be carried out in live cells such as cells, tissues, and organs. In the conventional screening method, mRNA, expressed due to the drug, is isolated, cDNA is prepared by reverse transcription reaction, amplified by PCR, amplified cDNA is labeled, and the emission of the label is detected (FIG. 7). This conventional screening method is very complicated and requires skillful expertise. However, in the method for screening a drug of the invention, the nucleic acid probe can be also introduced into live cells directly. Thus, screening can be carried out rapidly and easily.

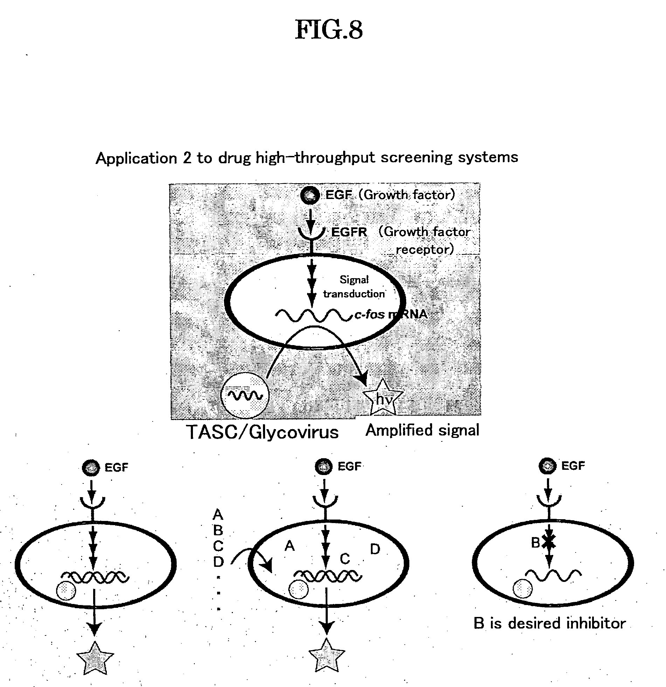

Further, the method for screening a drug of the invention is advantageous in that, as shown in FIG. 7, the use of live cells allows for the detection or analysis of behaviors of mRNA, etc., which could not be found because in the conventional method cells are fixed, and thus are not allowed to perform biological activity, and in addition allows for, e.g. detection or analysis of a trace amount of mRNA, etc. As shown in FIG. 8, a desired inhibitor can be also screened, and a new effect and/or side effect of an existing medicine can be found by detecting a trace amount of gene (presence or absence of target nucleic acid). Furthermore, the method can be applied to a doping test for athletes, narcotic detection test for addicts of narcotics, etc.

The drug, screened by the method for screening a drug of the invention, is not particularly limited and can be appropriately selected according to the purpose. Examples of the drug include drugs acting on the peripheral nerve, drugs acting on the central nervous system, hormone drugs, autacoids, drugs acting on the circulatory system, drugs acting on the respiratory system, drugs acting on the digestive system, drugs acting on the urogenital system, cutaneous drugs, vitamin compounds, chemotherapeutic agents, disinfectants, antiseptics, biological products, herbal medicines, and the like. The drug may be an existing medicine or new medicine.

Examples of the drug acting on the peripheral nerve include local anesthetics, muscle relaxants, drugs acting on the autonomic nervous system, and the like.

Examples of the local anesthetic include cocaine, tropacocaine, procaine, lidocaine, bupivacaine, mepivacaine, tetracaine, dibucaine, and the like.

Examples of the muscle relaxant include d-tubocurarine, gallamine, pancuronium, vecuronium, decamethonium, suxamethonium, dantroline, and the like.

Examples of the drug acting on the autonomic nervous system include epinephrine, norepinephrine, isoproterenol, dopamine, phenylephrine, methoxamine, clonidine, metaraminol, naphazoline, dobutamine, methoxyphenamine, orciprenaline, terbutaline, clorprenaline, trimetoquinol, salbutamol, tulobuterol, procaterol, pirbuterol, fenoterol, formoterol, clenbuterol, mabuterol, tyramine; ephedrine, methylephedrine, amphetamine, ergotamine, ergotoxine, ergometrine, dihydroergotoxine, phenoxybenzamine, tolazoline, phentolamine, prazosin, terazosin, doxazosin, bunazosin, urapidil, yohimbine, propranolol, bupranolol, bufetolol, bucumolol, nadolol, timolol, tilisolol, practlol, metoprolol, atenolol, bisoprolol, betaxolol, pindolol, oxprenolol, alprenolol, carteolol, indenolol, penbutolol, bunitrolol, bopindolol, acebutolol, celiprolol, labetalol, guanethidine, methyldopa, clonidine, guanfacine, guanabenz, acetylcholine, benatecol, methacholine, muscarine, pilocarpine, physostigmine, neostigmine, edrophonium, ambenonium, distigmine, parathion, atropine, scopolamine, scopolia extract, homatropine, cyclopentolate, nicotine, DMPP, TMA, DFP, TEPP, tetraethylammonium, hexamethonium, mecamylamine, trimethaphan, and the like.

Examples of the drug acting on the central nervous system include nitrous oxide, cyclopropane, ethylene, ether, chloroform, halothane, methoxyflurane, enflurane, isoflurane, sevoflurane, thiopental, thiamylal, hexobarbital, pentobarbital, propanidid, ketamine, phenobarbital, bromovalerylurea, bromodiethylacetylurea, chloral hydrate, glutethimide, metakwalon, nitrazepam, flurazepam, estazolam, triazolam, methylparafynol, ethchlorvynol, ethyl alcohol, methyl alcohol, disulfiram, phenytoin, mephenytoin, ethotoin, phenobarbital, metharbital, primidone, carbamazepine, zonisamide, trimethadione, paramethadione, sodium valproate, phensuximide, methsuximide, ethosuximide, diazepam, clonazepam, morphine, codeine, divine, papaverine, noscapine, opium tincture, codeine phosphate, heroin, pethidine, fentanyl, methadone, levorphanol, dextromethorphan, pentazocine, phenazocine, buprenorphine, butorphanol, nalorphine, levallorphan, naloxone, endorphin, mephenesin, eperisone, baclofen, tizanidine, levodopa, carbidopa, benserazide, trihexyphenidyl, biperiden, benztropine, amantadine, bromocriptine, pergolide, droxidopa, strychnine, picrotoxin, pentetrazole, nikethamide, dimorpholamine, caffeine, theophylline, theobromine, aminophylline, chlorpromazine, thioridazine, perphenazine, reserpine, rescinnamine, deserpidine, haloperidol, chlorprothixene, thiothixene, iproniazid, nialamide, isocarboxazid, safrazine, tranylcypromine, pargyline, imipramine, desipramine, amitriptyline, clomipramine, mianserin, lithium, diazepam, oxazepam, nitrazepam, etizolam, lorazepam, hydroxyzine, LSD-25, mescaline, cannabis, dimethyltryptamine, diethyltryptamine, psilocin, psilocybin, bufotenine, hamine, and the like.

The hormone drug is not particularly limited as long as it has or inhibits hormone action. Examples of the hormone drug include growth hormone, thyroid-stimulating hormone, adrenocorticotropic hormone, follicle-stimulating hormone, luteinizing hormone, lactogenic hormone, oxytocin, vasopressin, thyroid hormone, liothyronine sodium, levothyroxine sodium, methylthiouracil, propylthiouracil, thiamazole, calcitonin, parathormone, insulin, glucagon, carbutamide, chlorpropamide, acetohexamide, tolazamide, hexamide, 1-butyl-3-nitrourea, tolbutamide, glibenclamide, glymidine, glybuzole, phenformin, buformin, metformin, epalrestat, voglibose, acarbose, troglitazone, cortisone, hydrocortisone, desoxycortone, prednisolone, ethylprednisolone, paramethason, dexamethasone, betamethasone, aldosterone, estradiol, estrone, ethinylestradiol, stilbene, progestron, chlormadinone, norethisterone, testosterone, androsterone, etiocholanolone, epiandrosterone, methyltestosterone, testosterone aqueous suspension injection, testosterone ester injection, oxymetholone, methenolone, mixed hormone preparation, and the like.

Examples of the autacoid include histamine, diphenhydramine, diphenylpyraline, clemastine, pyrilamine, promethazine, chlorpheniramine, chlorcyclizine, cyproheptadine, mequitazine, terfenadine, cimetidine, ranitidine, famotidine, diphenhydramine, dimenhydrinate, cyclidine, meclizine, promethazine, thiethylperazine, serotonine, methysergide, proheptazine, rennin, angiotensin, captopril, prostaglandin E-2, prostaglandin F2α, prostaglandin A2, thromboxane A2, leukotriene, bradykinin, plasma kallikrein, glandular kallikrein, sodium salicylate, aspirin, salicylic amide, indomethacin, mefenamic acid, flufenamic acid, diclofenac, benzydamine, epirizole, tiaramide, phenacetin, acetaminophen, antipyrine, aminopyrine, sulpyrine, colchicines, probenecid, allopurinol, phenylbutazone, oxyphenbutazone, sulfinpyrazone, dexamethasone, betamethasone, aurothiomalate sodium, auranofin, penicillamine, azathioprine, bucillamine, salazosulfapyridine, actarit, and the like.

Examples of the drug acting on the circulatory system include digitalis, kedigitalis, strophanthus, sea onion, digitoxin, digoxin, methyldigoxin, G-strophanthin, lanatoside C, proscillaridin, ouabain, β-receptor agonist, amrinone, pimobendan, vesnarinone, quinidine, procainamide, disopyramide, mexiletine, aprindine, propafenone, flecainide, pilsicainide, adrenergic β-antagonist, amiodarone, verapamil, hydralazine, diazoxide, prazosin, reserpine, guanethidine, centrally acting drug, thiazide diuretic, captopril, enalapril, alacepril, delapril, cilazapril, lisinopril, benazepril, imidapril, temocapril, quinapril, trandolapril, amyl nitrite, nitroglycerine, isosorbide mononitrate, isosorbide dinitrate, pentaerythritol tetranitrate, molsidomine, nicorandil, dipyridamole, dicelap, prenylamine, chromonal, trimetazidine, oxyfedrine, etafenone, nifedipine, nicardipine, nisoldipine, nitrendipine, benidipine, manidipine, barnidipine, efonidipine, amlodipine, felodipine, diltiazem, verapamil, inositol nicotinate, cyclandelate, cinnarizine, isoxsuprine, alprostadil, meclofenoxate, vinpocetine, nicergoline, ibudilast, flunarizine, ifenprodil, pentoxifylline, idebenone, indeloxazine, bifemelane, propentofylline, nicotinic acid, cholestyramine, probucol, pravastatin, simvastatin, heparin, dicumarol, tolmexan, warfarin, urokinase, streptokinase, tissue plasminogen activator, ticlopidine, cilostazol, ethyl icosapentate, beraprost, thrombin, tranexamic acid, carbazochrome, iron sulfate, ferrous fumarate, sodium ferrous citrate, saccharated iron oxide, erythropoietin, epoetin alfa, epoetin beta, filgrastim, lenograstim, nartograstim, mirimostim, sildenafil citrate, and the like.

Examples of the drug acting on the respiratory system include carbon dioxide, dimorpholamine, dimefline, doxapram, saponins, nonsaponin glycoside, potassium iodide, bromhexine, methyl cysteine, ethyl cysteine, acetylcysteine, carbocysteine, ambroxol, xanthine derivative, cromolyn sodium, ketotifen, tranilast, azelastine, oxatomide, ozagrel, suplatast tosylate, terfenadine, astemizole, seratrodast, pranlukast hydrate, and the like.

Examples of the drug acting on the digestive system include sodium hydrogencarbonate, magnesium oxide, natural aluminum silicate, dried aluminum hydroxide gel, parasympatholytic, pirenzepine, tiquizium, cimetidine, famotidine, ranitidine, omeprazole, lansoprazole, methylmethionine sulfonium chloride, azulene sulfonate, sucralfate, aldioxa, gefarnate, teprenone, troxipide, irsogladine, sulpiride, sofalcone, plaunotol, rebamipide, benexate betadex, ornoprostil, misoprostol, enprostil, clarithromycin, amoxicillin, metronidazole, bismuth subnitrate, ricinus, anthraquinone derivative, phenolphthalein, picosulfate, magnesium sulfate, magnesium oxide, sodium sulfate, glycerin, bisacodyl, albumin tannate, precipitated calcium carbonate, medicinal carbon, opium alkaloid, berberine, lactomin, metoclopramide, domperidone, cisapride, trimebutine, and the like.

Examples of the drug acting on the urogenital system include D-marinitol, concentrated glycerin, ammonium chloride, ammonium nitrate, calcium chloride, mercurial diuretics, acetazolamide, dichlorphenamide, trichlormethiazide, hydrochlorothiazide, benzylhydrochlorothiazide, penflutizide, methyclothiazide, metolazone, chlorthalidone, furosemide, ethacryic acid, piretanide, bumetanide, azosemide, mefruside, spironolactone, triamterene, potassium canrenoate, hexamine, mandelic acid, nitrofurantoin, ergotoxine, ergotamine, ergometrine, methylergometrine, oxytocin, dinoprost, dinoprostone, gemeprost, sparteine, phenylmercuric acetate, oxyquinoline sulfate, oral contraceptive, and the like.

Examples of the cutaneous or mucosal drugs include mustard, cantharis, red pepper, tannic acid, gallic acid, zinc oxide, lead monoxide, lead tetroxide, zinc sulfate, lead acetate, aluminum sulfate, aluminum potassium sulfate, sodium hydroxide, potassium hydroxide, sodium carbonate, potassium carbonate, glacial acetic acid, trichloroacetic acid, hydrochloric acid, lactic acid, silver nitrate, ferric chloride, zinc chloride, copper sulfate, copper citrate, salicylic acid, sulfur, gum arabic, tragacanth gum, starch, agar, methylcellulose, glycerin, propylene glycol, olive oil, camellia oil, sesame oil, rapeseed oil, soybean oil, peanut oil, corn oil, coconut oil, cacao butter, stearic acid, beef tallow, lard, lanolin, paraffin, petrolatum, bees wax, talc, kaolin, lycopodium, medicinal carbon, fibrinogen, thromboplastin, absorbable gelatin sponge, oxycellulose, methoxsalen, monobenzone, and the like.

Examples of the vitamin compound include vitamin A, vitamin D, vitamin E, vitamin F, vitamin K, ubiquinone, thiamine, riboflavin, pyridoxine, pantothenic acid, α-lipoic acid, biotin, folic acid, mezoinotitol, p-aminobenzoic acid, cyanocobalamin, prosultiamine, fursultiamine, octotiamine, bisbentiamine, benfotiamine, shikotiamine, dicethiamine, ascorbic acid, vitamin P, and the like.

Examples of the chemotherapeutic agent include antibiotics such as penicillins, cephems, oxacephems, monobactams, carbapenems, βlactamase inhibitor, fosfomycin, aminoglycosides, chloramphenicol, tetracyclines, macrolides, lincomycins, rifamycin, antimycobacterium agents, polyene macrolides, and systhetic antibacterial agents; sulfa drugs, quinolone drugs, vidarabine, idoxuridine, acyclovir, ganciclovir, zidovudine, didanosine, interferon, amantadine, emetine, pyrimethamine, chloroquine, primaquine, quinacrine, metronidazole, pentamidine isetionate, santonin, kainic acid, piperazine salts, pyrantel pamoate, praziquantel, kamala, antimony sodium tartrate, diethylcarbamazine, hexylresorcin, antimonial, niclosamide, biotinol, bephenium hydroxynaphthoate, pyrvinium pamoate, isothiocyanide, streptomycin, rifampicin, ethambutol, para-aminosalicylic acid, mechlorethamine hydrochloride, cyclophosphamide, melphalan, thiotepa, busulfan, nimustine, methotrexate, 6-mercaptopurine, 5-fluorouracil, cytarabine, diethylstilbestrol, ethinylestradiol, testosterone propionate, fluoxymesterone, clausterone, hydrocortisone, prednisolone, prednisone, dexamethasone, tamoxifen citrate, toremifene citrate, actinomycin D, anthracycline, doxorubicin, daunorubicin, aclarubicin, epirubicin, pirarubicin, bleomycin, peplomycin, mitomycin C, vinblastine, vindesine, etoposide, irinotecan, L-asparaginase, pentostatin, cisplatin, tretinoin, picibanil, krestin, oseltamivir phosphate, and the like.

Examples of the biological products include a diphtheria tetanus combined vaccine, oral poliomyelitis vaccine, dried live attenuated rubella vaccine; dried live attenuated measles vaccine, influenza HA vaccine, Japanese encephalitis vaccine, dried BCG vaccine, variolovaccine, cholera vaccine, Weil's disease and Akiyami combined vaccine, tetanus toxoid, diphtheria toxoid, gas gangrene antitoxin, diphtheria antitoxin, snake antitoxin, tetanus antitoxin, purified tuberculin, BCG, and the like.

Examples of the herbal medicine include Anchusan, Chotosan, Orengedokuto, Keishibukuryogan, Shosaikoto, Yokukansan, Hachimijiogan, Kakkonto, and the like.

### (Apparatus for detecting target nucleic acid)

As the apparatus for detecting a target nucleic acid of the invention, the following first and second apparatuses for detecting a target nucleic acid are suitable.

The first apparatus for detecting a target nucleic acid is a device for analyzing a nucleic acid in which a nucleic acid probe for detecting a target nucleic acid in a sample is immobilized on a support. The nucleic acid probe is designed so that it undergoes a conformational change after hybridization to the target nucleic acid, the nucleic acid probe, which underwent the conformational change, dissociates from the target nucleic acid as a result of the decrease of the binding strength of hybridization.

The second apparatus for detecting a target nucleic acid comprises a unit configured to allow, or hybridization means for allowing, the nucleic acid probe of the invention to hybridize to a target nucleic acid; and a target nucleic acid detector configured to detect the presence of the target nucleic acid by detecting the signal generated from the nucleic acid probe upon hybridization to the target nucleic acid.

The hybridization means is not particularly limited as long as it allows the nucleic acid probe of the invention to hybridize to the target nucleic acid, and can be appropriately selected according to the purpose. Suitable examples include the nucleic acid chip of the invention, and the like.

The target nucleic acid detector is a means for detecting the target nucleic acid by detecting the signal generated when the nucleic acid probe of the invention has hybridized to the target nucleic acid. For example, when the above-described signal is emission, quenching, etc., it is detected by a photodetector, camera, etc. In the case of emission of radiation, it is detected by a sensitive film, etc. In the case of temperature change, by a thermocouple, temperature sensor, etc. In the case of change in magnetic force, by a magnetometer. In the case of generation of a cleaved fragment, by an electrophoresis, SDS-PAGE, western blotting, etc. In the case of substance production, by an antibody, HPLC, affinity chromatography, etc. In the case of substance consumption, by an IR spectrum, MS spectrum, etc. In the case of substance consumption, by an IR spectrum, MS spectrum, etc. In the case of deformation, by an electron microscope, etc. In the case of viscosity change, by a viscosity sensor, etc. These may be used alone or two or more may be used in combination.

The first and second probe fragments are generated by the cleavage of the nucleic acid probe when the nucleic acid probe has hybridized to the target nucleic acid. When the signal is emission of the light-emitting portion which is present in the first probe fragment, suitable examples of the target detector include a fluorescence microscope, sensitive film, CCD camera, and the like.

In the invention, it is preferable that the target nucleic acid detector can detect not only the presence of the target nucleic acid, but also the amount thereof quantitatively.

Suitable examples of the other means include a database part which stores database of genomic information, a data analysis part which compares and analyzes the data of the result of detection by the target nucleic acid detector and the genomic information data, which are stored in the database part, a communication part capable of communicating with the internet, and capable of accessing to database of genomic information on the internet, a data analysis part which compares and analyzes the data of the result of detection by the target nucleic acid detector and the genomic information data of the database of genomic information on the internet, and the like.

The database of genomic information is not particularly limited, can be appropriately selected according to the purpose, but is preferably those containing genomic information of healthy individuals and patients, related to particular diseases.

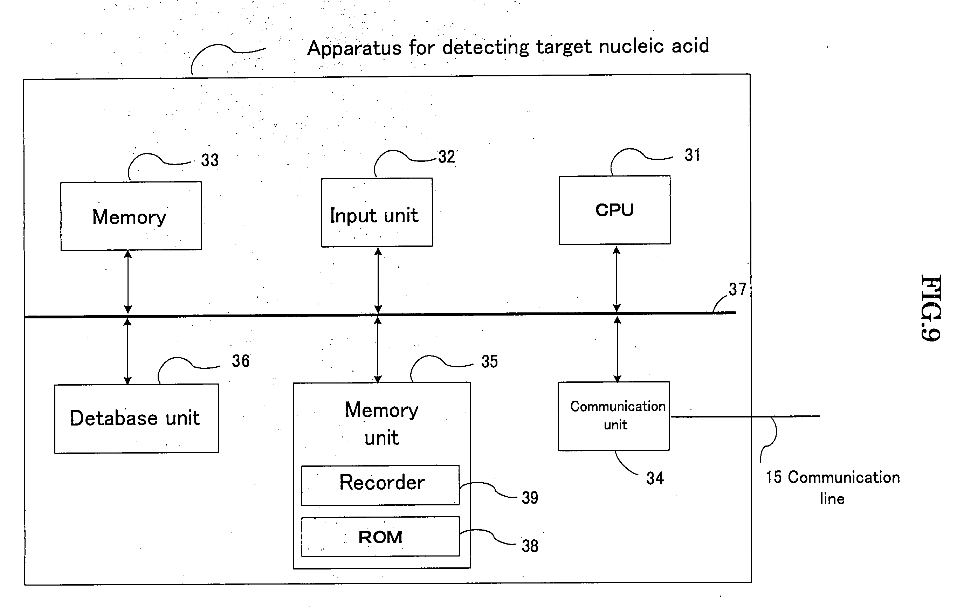

Suitable specific example of the apparatus for detecting a target nucleic acid of the invention include the apparatus for detecting a target of the invention comprising the nucleic acid chip of the invention as the hybridization means and the fluorescence microscope as the target detector, wherein apparatus for detecting a target further includes a CPU 31, input unit 32, memory 33, communication unit 34, database unit 36, and main bus 37 connecting these so as to allow two-way communication (FIG. 9).

The CPU 31 controls the entire of the apparatus for detecting a target nucleic acid, works based on the program stored in the memory 33, various kinds of data, etc, realizing a variety of functions. Here, the variety of functions include a remote diagnosis function which diagnoses data received from other apparatuses for detecting a target nucleic acid, a verification processing function which unionizes different signals in order to control the passing of information between other providers and other servers, a database processing function which provides a variety of information related to the target nucleic acid, and the like. The various kinds of data include date of various kinds of lists prepared for gene diagnosis.

The input unit 32 is a device by which an administrator of provider inputs e.g. parameters of gene diagnosis. The memory 33 has a function of storing a program for being accessed by the CPU 31, the database of genomic information, information which the apparatus for detecting a target nucleic acid has received, information read out from a memory unit 35, the database unit 36, etc. according to necessity. The communication unit 34 is a unit controlling communication with the outside through the internet, communication line 15, etc. The communication unit 34, for example, may comprise a modem as a conversion unit, and may comprise a terminal adapter (TA) which lies for connecting an ISDN line, and digital service unit (DSU).

The memory unit 35 stores a variety of programs and data, has a function of memorizing, and comprises, for example, a ROM 38 as a memorizing means and a recorder 39 which reads storage media memorized in the ROM 38. The memory unit 35 includes an electronic memory circuit (electronic storage media) such as a ROM, and besides, a magnetic storage device in which magnetic storage media such as floppy-disks are the storage media, an optical storage device in which optical recording media such as CD-ROMs are the storage media, and the like. The storage media may be those provided fixed to the memory unit 35, or may be those provided detachably. The programs, data, etc. to be memorized in the memory unit 35 may be received via communications means from the memory device outside the apparatus for detecting a target nucleic acid.

The database unit 36 stores a list of past data of the target nucleic acid, etc. inside thereof.

### (Gene diagnosis method)

The gene diagnosis method of the invention uses the apparatus for detecting a target gene of the invention, and comprises a target nucleic acid expression level quantitative determination step and a diagnosis step, and may further comprise other steps appropriately selected according to necessity.

The target nucleic acid expression level quantitative determination step is a step in which the expression level of a target nucleic acid related to a specific disease in a subject is determined quantitatively.

The step is suitably carried out by the method for detecting a target nucleic acid of the invention using the apparatus for detecting a target nucleic acid of the invention described above. For example, the step can be carried out by preparing beforehand a standard curve representing the relationship between the expression level of the target nucleic acid and emission level using the nucleic acid probe of the invention, and by determining the quantity using the standard curve.

The diagnosis step is a step in which the expression level of the target nucleic acid related to the specific disease in the subject, and the expression levels, in a healthy individual and in a patient, of the target nucleic acid related to the specific disease which are contained in a database of genomic information, are compared by a data analysis part to diagnose whether or not the subject is a patient of the specific disease.

The diagnosis step can be suitably carried out by the database part, data analysis part, communication part, data analysis part, and the like in the apparatus for detecting a target of the invention. The diagnosis step is carried out, for example, by allowing the memory unit 35 described above to memorize the program of the gene diagnosis method, allowing the database unit 36 to memorize S value and M value of the (Xi-S/M-S) described later, which are averaged from the past detection data, determining the disease-related gene (the Xi) of the subject quantitatively with the apparatus for detecting a target nucleic acid, and allowing the CPU to calculate the (Xi-S/ M-S) and diagnose health condition of the subject. The gene diagnosis method of the invention is advantageous in that diagnosis can be carried out even in a remote area, etc.

In the diagnosis step, for example, the subject may be diagnosed according to the following calculation. Specifically, if (Xi-S/M-S) ≥ 0.9 is satisfied, a subject is diagnosed as a patient of a specific disease, where M: expression level of a target gene related to the specific disease, of the patient, S: expression level of a healthy individual, and Xi: expression level of the subject. Such calculation can be carried out by the CPU, etc. of the apparatus for detecting a target nucleic acid instantly. This enables fast computer diagnosis.

If the value (Xi-S/M-S) is less than or equal to 0.1, it normally means that one does not have factors of a disease, or has a good prognosis, as the value approaches to 0.9, it means that expression of the target nucleic acid (disease-related gene) of a subject approaches to the condition of a specific disease, and if the value exceed 0.9, it means that the expression level of the target nucleic acid (disease-related gene) is 90% close to those of a patient having the disease-related gene and of a healthy individual having the factor.

The other step is not particularly limited and can be appropriately selected according to the purpose. Examples thereof include a selection step in which a disease-related gene is selected from candidates, and the like.

The gene diagnosis method of the invention can diagnose any disease or disorder without limitation, and the method can be appropriately selected according to the purpose. Examples thereof include cancer, alkaptonuria, systemic lupus erythematosus, dermatomyositis, nic rheumatism, scleroderma, psychosis, bronchial asthma, atopic dermatitis, pollinosis, lupus nephritis, Creutzfeldt-Jakob disease, Alzheimer disease, Parkinson's disease, Marfan's syndrome, Williams-syndrome, hypoplastic anemia, erythrocytosis, Fanconi's syndrome, hemophilia A, hemophilia B, von Willebrand's disease, thrombasthenia, Idiopathic thrombocytopenic purpura, Chediak-Higashi syndrome, pemphigus vulgaris, Felty syndrome, fragile X syndrome, Edward syndrome, Miller-Decker syndrome, Prader-Willi syndrome, Bloch-Sulzberger syndrome, Lowe syndrome, Klinefelter's syndrome, Angelman syndrome, retinitis pigmentosa, nail-patella syndrome, Behcet's syndrome, Machado-Joseph dsease, Zollinger-Ellison syndrome, cat-cry syndrome, Turner's syndrome, storage pool disease, Melkersson-Rosenthal syndrome, aldosteronism, Hashimoto's disease, cretinism, Cushing's syndrome, syndrome of inappropriate antidiuretic hormone, acromegaly, sickle cell anemia, ulcerative colitis, Crohn's disease, familial polyposis of the colon, Down's syndrome, diabetes, maple syrup disease, amyotrophic lateral sclerosis, muscular dystrophy, gout, polymyositis, Sjogren's syndrome, mixed connective tissue disease, Pierre Robin syndrome, Plummer-Vinson syndrome, Ramsay Hunt syndrome, facial hemiatrophy, ectodermal dysplasia, dysosteogenesis, Osler's disease, Reiter's syndrome, sarcoidosis, juvenile rheumatoid arthritis, Gilbert syndrome, Rotor's syndrome, hemochromatosis, phenylketonuria, glycinuria, tyrosinosis, cystinuria, homocystinuria, Horner's syndrome, Prader-Willi syndrome, Gaucher's disease, histidinemia, Wilson disease, osteomalacia, cystic fibrosis, abnormal porphyrin metabolism, abnormal bilirubin metabolism, adenosine deaminase deficiency, autoimmune hemolytic anemia, thalassemia, hemoglobin C disease, pyruvate kinase deficiency, G6PD deficiency, hereditary spherocytosis, familial periodic paralysis, myasthenia gravis, Guillain-Barre syndrome, multiple sclerosis, hypoparathyroidism hyperparathyroidism, Clauson disease, insulinoma, Basedow's disease, hyperlipemia, chorea, high blood pressure, glaucoma, cataract, and the like. The gene diagnosis method of the invention not only can diagnose these diseases or disorders, but also can assess prognosis of these diseases or disorders, or risk of suffering from diseases. The gene diagnosis method also can, for example, analyze adaptability, etc. of a living body to an administered drug by expression level of a target nucleic acid (gene).

Examples of the invention will be described below, but the invention is not restricted to these Examples.

### (Example 1)

### - Design of nucleic acid probe -

As the nucleic acid probe of the invention, a base sequence was used that comprises base sequences 3a and 3b, regions capable of hybridizing to the target nucleic acid, at both ends, and comprises a ribozyme known in the art (8 to 17 DNAzyme) at the portion thereof. The base sequence had a base sequence shown in the following sequence 1, and "rA" as the cleavage portion was located at the 15th position from the 5' end. Further, the cleavage active region (exhibits ribozyme activity) having activity to cleave "rA" as the cleavage portion was present between T located at the 32nd from the 5' end and A located at the 46th from the 5' end. In this selected base sequence, thymine (dT) to which a fluorescent substance (F): fluorescein as the light-emitting portion bound, i.e., fluorescein-dT (available from Glen Research Corporation) was used as the 14th base (T (dT)) from the 5' end, and thymine (dT) to which a quenching substance (Q): dimethylaminobenzenesulfonyl (DABSYL) as the quenching portion bound, i.e., dimethylaminobenzenesulfonyl (DABSYL)-dT (available from Glen Research Corporation) was used as the 18th base (T) from the 5' end. In this way, the nucleic acid probe of Example 1 was prepared. The range from A, the 1st from the 5' end, to C, the 10th from the 5' end was designated as 3b, and the range from C, the 1st from the 3' end, to C, the 10th from the 3' end was designated as 3a.

(Sequence 1)

5′-ACTTCTCATCAACTrAGGTCTTTTTTTTTGACTCCGAGCC

GGACGAAGTTCTAGTTGGTC-3′

FIG. 2 is a schematic diagram of the one-dimensional structure of the nucleic acid probe of Example 1, and the base sequence 3a side is the 3' end side and the base sequence 3b side is the 5' end side (left/right is reversed compared to the above (Sequence 1)). As shown in FIG. 2, the "rA" as the cleavage portion is present between the light-emitting portion and the quenching portion. Therefore, when the cleavage portion is cleaved and the nucleic acid probe of Example 1 is divided into first and second probe fragments, the light-emitting portion is present on the first probe fragment, and the quenching portion is present on the second probe fragment.

FIG. 3 is a schematic diagram of the three-dimensional structure when the nucleic acid probe of Example 1 has hybridized to a target nucleic acid. As shown in FIG. 3, there were 2 regions capable of hybridizing intramolecularly upon hybridization to the target nucleic acid, those being located adjacent to each other with the cleavage portion centered (via the cleavage portion). Of the two regions, the region close to the base sequences 3a and 3b was designated as Y and the other was as Z. The number of bases in the sequence of the Y was 4, the number of bases in the sequence of the Z was 4, and the total number of bases in the sequences of the Y and Z was 8.

### - Synthesis of nucleic acid probe -

The nucleic acid probe of Example 1 designed as described above was solid phase synthesized by a solid-phase synthesis using a DNA synthesizer, deprotection was carried out, and only the nucleic acid probe with a chain length of interest was separated and purified by a gel electrophoresis.

Initially, light-emitting substance F and quenching substance Q were protected using A-TOM-CE (phosphoramidites) (available from Glen Research Corporation) as a protecting group, and a nucleic acid probe of the invention, in which the labeling substance with a concentration of 1 µmol was introduced, was synthesized by a solid phase method using the DNA synthesizer (available from Glen Research Corporation).

After the synthesis, the product was transferred to a tube, and to the tube, 1.5 ml of methylamine (mixture of equal amounts of 40% methylamine aqueous solution and 33% ethanolic methylamine solution) was added, incubated at 35°C for 6 hours, the nucleic acid probe was taken out from the solid phase, and the protecting group of the labeling substance was removed.

Next, the tube was cooled in ice, then the tube was opened carefully, supernatant was collected under sterile condition, and freeze-dried. After the freeze-dry, to the tube, 1ml of tetrabutylammonium solution (1 M in tetrahydrofuran (THF)) was added, shaked at 50°C for 10 minutes, the protecting group attached to the nucleic acid probe and a group at the site of the labeling substance were removed, cooled to 35°C, and then shaked again for 6 hours.

Next, 1 ml of 1M Tris buffer solution (pH 7.4) was added to the tube, then shaked well, and the THF was removed by a centrifugal concentration method. Further, to the tube, 1 ml of 1 M Tris buffer solution (pH 7.4) was added, and the total amount of the nucleic acid probe solution in the tube was adjusted to 2 ml.

### - Purification of nucleic acid probe -

A NAP-25 column (available from Pharmacia Corporation) was equilibrated with 15 ml of water, and then to the NAP-25 column, 2 ml of the nucleic acid probe solution was added. The nucleic acid probe solution was allowed to penetrate into the column completely, then 0.5 ml of distillated water was added, so-called gel filtration was carried out, and impurity was removed. Further, the desalted nucleic acid probe was eluted using 3 ml of distillated water.

The nucleic acid probe after the gel filtration was freeze-dried, and then, was purified by 7 M urea-containing 20% polyacrylamide gel electrophoresis. After the electrophoresis, the band of the nucleic acid probe was confirmed by UV irradiation, and the fraction of the band was cut out. The fraction was put in a centrifuging tube, gel was crushed using a glass rod, 10 ml of water was added, and shaked at 37°C for 6 hours. After dialysis, freeze dry treatment was carried out and solid object was obtained. The solid object was dissolved into 0.4 ml of distillated water, and then refrigerated at -20°C.

### - Hybridization and cleavage active reaction -

As the target nucleic acid, 22 nt oligonucleotide having a base sequence shown in the following Sequence 2 was prepared.
(Sequence 2)
5'-GACCAACTAGAAGATGAGAAGT-3'

500 pmol of the target nucleic acid and 100 pmol of the nucleic acid probe were incubated in 100 µl of an aqueous solution of 50 mM Tris-25 mM magnesium chloride (pH 7.2) at 37°C for 1.5 hours. Further, 800 µl of ethanol was added, centrifugation was carried out at 4°C and 12,000 g, and ethanol precipitation was carried out. Supernatant was removed, and remaining ethanol was completely removed by vacuum drying.

### - Detection of target nucleic acid -

Precipitate, from which the ethanol was completely removed by the vacuum drying, was dissolved with 5 µl of formaldehyde, and electrophoresis was carried out using 7 M urea-containing 20% polyacrylamide gel. When irradiated with a UV lamp to confirm the presence of bands, the nucleic acid probe fragment, shorter than the nucleic acid probe in length, was observed. As a result, it was confirmed that the nucleic acid probe hybridized with the target nucleic acid, and self-cleavage of the cleavage portion by the cleavage active region occurred.

10 pmol of the target nucleic acid and 100 pmol of the nucleic acid probe were incubated in 100 µl of an aqueous solution of 50 mM Tris-25 mM magnesium chloride (pH 7.2) at 37°C for 6 hours (the concentration of magnesium ion was 25 mM). This reaction mixture was irradiated with exciting light with a wavelength of 495 nm, and fluorescence spectral change at a fluorescence wavelength of 520 nm for measurement was measured using a spectrofluorophotometer (RF-5300PC, available from Shimadzu Corporation). From the increase of fluorescence intensity, it was confirmed that the nucleic acid probe hybridized with the target nucleic acid, and self-cleavage of the cleavage portion by the cleavage active region occurred.

FIG. 4 is a graph which was prepared by determining the cleavage amount of the nucleic acid probe from the change in emission intensity. In FIG. 4, the continuous line represents the cleavage amount of the nucleic acid probe determined from the intensity of the emission, and the doted line represents a theoretical value in the case where a conventional nucleic acid probe, by which amplification of emission is impossible, was used.

From this result, it was found that in the case of the conventional nucleic acid probe, only one equivalent amount of probe reacted with one equivalent amount of the target nucleic acid, however, in the case of the nucleic acid probe of the invention, the cleavage amount increased with time, one or more equivalent amount of probes reacted with one equivalent amount of the target nucleic acid, resulting in the increase of emission.

### (Example 2)

### - Design of nucleic acid probe -

As the nucleic acid probe of the invention, nucleic acid probes having base sequences of the following Sequences 3 to 5 were selected. Specifically, a single-strand DNA random sequence pool was prepared that comprises at both ends a X side sequence (GTAGGAGT from the 5' end side) following the 3' end side of a X sequence located in the 5' end side, and a Y side sequence (GTGCCAGG from the 3' end side) following the 5' end side of a Y sequence located in the 3' end side, those sequences being the regions capable of hybridizing to the target nucleic acid. The DNA random sequence pool contains a large number of base sequences of which random sequences between the X side sequence (number of bases in the sequence: 8) and the Y side sequence (number of bases in the sequence: 8) are different in length or arrangement. The DNA random sequence pool was synthesized using a DNA synthesizer as follows. Specifically, a random sequence following the Y side sequence was synthesized by extending a sequence containing the Y sequence located in the 3' end side and the Y side sequence by one base from cytosine (C) located in the 5' end side of the Y side sequence, and to the 5' end side of the random sequence, guanine (G) in the 3' side of the sequence where the 3' end of the X sequence and the 5' end of the X side sequence were bound was allowed to bind. This random sequence comprises one "rA" (base is adenine and sugar is ribose) as the cleavage portion.

Next, base sequences with high cleavage activity at the cleavage portion were selected among the large number of base sequences contained in the DNA random sequence pool. These selected base sequences had base sequences shown in the following Sequences 3 to 5

In Sequence 3, the rA as the cleavage portion was located at the 14th position from the 5' end. Further, the cleavage -active region (exhibits ribozyme activity) having activity to cleave "rA" as the cleavage portion was present between T located at the 29th position from the 5' end and A located at the 43rd position from the 5' end. In Sequence 3, thymine (dT) to which a quenching substance (Q): dimethylaminobenzenesulfonyl (DABSYL) as the quenching portion bound, i.e.,: dimethylaminobenzenesulfonyl (DABSYL)-dT (available from Glen Research Corporation) was used as the 22nd base (T (dT)) from the 5' end, and a fluorescent substance (F): fluorescein as the light-emitting portion was allowed to bind to the 5' end, i.e., fluorescein (available from Glen Research Corporation) was allowed to bind. In this way, the nucleic acid probe of Sequence 3 in Example 2 was prepared.

In Sequence 4, the rA as the cleavage portion was located at 20th position from the 5' end. Further, the cleavage active region (exhibits ribozyme activity) having activity to cleave "rA" as the cleavage portion was present between T located at the 35th position from the 5' end and A located at the 49th position from the 5' end. In Sequence 4, thymine (dT) to which a quenching substance (Q): dimethylaminobenzenesulfonyl (DABSYL) as the quenching portion bound, i.e., dimethylaminobenzenesulfonyl (DABSYL)-dT (available from Glen Research Corporation) was used as the 28th base (T (dT)) from the 5' end, and a fluorescent substance (F): fluorescein as the light-emitting portion was allowed to bind to the 5' end, i.e., fluorescein (available from Glen Research Corporation) was allowed to bind. In this way, the nucleic acid probe of Sequence 4 in Example 2 was prepared.

In Sequence 5, the rA as the cleavage portion was located at 18th position from the 5' end. Further, the cleavage active region (exhibits ribozyme activity) having activity to cleave "rA" as the cleavage portion was present between T located at the 33rd position from the 5' end and A located at the 47th position from the 5' end. In Sequence 5, thymine (dT) to which a quenching substance (Q): dimethylaminobenzenesulfonyl (DABSYL) as the quenching portion bound, i.e., dimethylaminobenzenesulfonyl (DABSYL)-dT (available from Glen Research Corporation) was used as the 26th base (T (dT)) from the 5' end, and a fluorescent substance (F): fluorescein as the light-emitting portion was allowed to bind to the 5' end, i.e., fluorescein (available from Glen Research Corporation) was allowed to bind. In this way, the nucleic acid probe of Sequence 5 in Example 2 was prepared.

(Sequence 3)

5′-CCGTAGGAGTGCTrAGTCTTTTTTTTTGATCCGAGCCGG

ACGAAGCGGACCGTGTC-3′

(Sequence 4)

5′-AAAAAGCCGTAGGAGTGCTrAGTCTTTTTTTTGATCCG

AGCCGGACGAAGCGGACCGTGTC-3′

(Sequence 5)
5′-AAAAAGGTAGGAGTGCTrAGTCTTTTTTTTTGATCCGAG
CCGGACGAAGCGGACCGTG-3′

FIG. 5B is a schematic view of the one-dimensional structure of the nucleic acid probe of Example 2.

As shown in FIG. 5A, in the nucleic acid probe of Sequence 3, the X sequence in the 5' end side was 2 bases, CC, from the 5' end side, and the Y sequence in the 3' end side was 2 bases, CT, from the 3' end side, and thymine (T(dT)) to which dimethylaminobenzenesulfonyl (DABSYL), a quenching substance Q, bound, i.e., dimethylaminobenzenesulfonyl (DABSYL)-dT (available from Glen Research Corporation) was used as the base (T) located at *mark, and a phosphate group to which fluorescein, a fluorescent substance F, bound, i.e., fluorescein phosphoramidite (available from Glen Research Corporation) was used as the base located at 5' end (furthest end). In the nucleic acid probe of Sequence 3, the number of bases in the sequence of the regions which hybridize with the target nucleic acid was 10 in the 3' end side, and was 10 in the 5' end side.

As shown in FIG. 5A, in the nucleic acid probe of Sequence 4, the X sequence in the 5' end side was 8 bases, AAAAAGCC, from the 5' end side, and the Y sequence in the 3' end side was 2 bases, CT, from the 3' end side, and thymine (T(dT)) to which dimethylaminobenzenesulfonyl (DABSYL), a quenching substance Q, bound, i.e., dimethylaminobenzenesulfonyl (DABSYL)-dT (available from Glen Research Corporation) was used as the base (T) located at *mark, and a phosphate group to which fluorescein, a fluorescent substance F, bound, i.e., fluorescein phosphoramidite (available from Glen Research Corporation) was allowed to bind to 5' end (furthest end). In the nucleic acid probe of Sequence 3, the number of bases in the sequence of the regions which hybridize with the target nucleic acid was 10 in the 3' end side, and was 10 in the 5' end side.

As shown in FIG. 5A, in the nucleic acid probe of Sequence 5, the X sequence in the 5' end side was 6 bases, AAAAAG, from the 5' end side, and the Y sequence in the 3' end side was 0 base, and thymine (T(dT)) to which dimethylaminobenzenesulfonyl (DABSYL); a quenching substance Q, bound, i.e., dimethylaminobenzenesulfonyl (DABSYL)-dT (available from Glen Research Corporation) was used as the base (T) located at *mark, and a phosphate group to which fluorescein, a fluorescent substance F, bound, i.e., fluorescein phosphoramidite (available from Glen Research Corporation) was allowed to bind to 5' end (furthest end). In the nucleic acid probe of Sequence 5, the number of bases in the sequence of the regions which hybridize with the target nucleic acid was 8 in the 5' end side, and was 8 in the 3' end side.

The nucleic acid probes of Sequences 3 to 5 in Example 2 may be referred to as "TASC 1 probe", "Locked TASC 2 probe", and "Locked TASC 3 probe", respectively.

As shown in FIG. 5A, in each of the nucleic acid probes of Sequences 3 to 5, there were 2 regions capable of hybridizing intramolecularly upon hybridization to the target nucleic acid, those being located adjacent to each other with the cleavage portion (rA) centered (via the cleavage portion). Of the two regions, the region close to the X sequence and Y sequence was designated as Y and the other was as Z. The number of bases in the sequence of the Y was 3, the number of bases in the sequence of the Z was 3, and the total number of bases in the sequences of the Y and Z was 6. Further, in each of the nucleic acid probes of Sequences 3 to 5, the cleavage active region (exhibits ribozyme activity) was formed in the side facing the cleavage portion (rA) upon hybridization to the target nucleic acid, and the cleavage active region contained a 3-base-pair complementary strand portion.

Before hybridization to the target nucleic acid, each of the nucleic acid probes of Sequences 3 to 5 had a stem loop structure as shown in the right of FIG. 5A due to intramolecular hybridization of the X sequence, located at the 5' end (5' furthest end), and a complementary sequence to the X sequence, located at the position not at the furthest end but the position away from the furthest end to the 5' end side in the 3' end side. This stem loop structure was unbound when the nucleic acid probe has hybridized to the target nucleic acid. The nucleic acid probe of Example 2 was the Locked TASC probe, kept the stem loop structure and the cleavage active region was not formed before hybridization to a target nucleic acid. Only when the stem loop structure was unbound upon hybridization to the target nucleic acid, the cleavage active region was formed.

### - Synthesis of nucleic acid probe -

The nucleic acid probe designed as described above was solid phase synthesized by a solid-phase synthesis using a DNA synthesizer, deprotection was carried out, and only the nucleic acid probe with a chain length of interest was separated and purified by a gel electrophoresis as follows.

Initially, the nucleic acid probes were each synthesized with a DNA/RNA synthesizer (ABI 392, available from PerkinElmer Inc.) using Bz-dA, Bu-dG, Ac-dC, which are phosphoramidites (available from Glen Research Corporation), fluorescein, Ac-A-Tom, and dabsyl-dT.

After the synthesis, synthesized product was transferred to a tube, 1.5 ml of mixture of equal amounts of methylamine solution (40% methylamine aqueous solution (75590, available from Fluka Chemical Co.) and 33% ethanolic methylamine solution (75580, available from Fluka Chemical Co.) was added, incubated at 35°C for 6 hours, and impurity attached to the nucleic acid probe was removed.

The tube was cooled in ice, then the tube was opened carefully, supernatant was collected under sterile condition, freeze-dried. After the freeze-dry, to the tube, 1ml of tetrabutylammonium solution (1M in tetrahydrofuran (THE)) was added, shaked at 50°C for 10 minutes, cooled to 35°C, and then shaked again for 6 hours. Thus, the protecting group attached to the nucleic acid probe was removed.

### - Purification of nucleic acid probe -

A NAP-25 column (available from Pharmacia Corporation) was equilibrated with 15 ml of distillated water, and then to the NAP-25 column, 2 ml of the nucleic acid probe solution was added. The nucleic acid probe solution was allowed to penetrate into the column completely, then 0.5 ml of distillated water was added, so-called gel filtration was carried out, and impurity was removed. Further, the desalted nucleic acid probe was eluted using 3 ml of distillated water.

The nucleic acid probe after the gel filtration was freeze-dried, and then, was purified by 7 M urea-containing 8% polyacrylamide gel electrophoresis. After the electrophoresis, the band of the nucleic acid probe was confirmed by UV irradiation, and the fraction of the band was cut out. The fraction was put in a centrifuging tube, gel was crushed using a glass rod, 10 ml of water was added, and shaked at 37°C for 6 hours. After dialysis; freeze dry treatment was carried out and solid object was obtained. The solid object was dissolved into 0.4 ml of distillated water, and then refrigerated at -20°C.

### - Hybridization and cleavage active reaction -

As the target nucleic acid, 22 nt oligonucleotide shown in the following Sequence 6 and 22 nt oligonucleotide shown in the following Sequence 7 were prepared. These oligonucleotides are RNA sequence of 16S ribosomal RNA 326-347 region in *E. Coli* K12-MG1655, and its DNA sequence in which RNA was displaced with DNA, respectively.
(Sequence 6)
5'-GACACGGTCCAGACTCCTACGG-3'
(Sequence 7)
5'-GACACGGUCCAGACUCCUACGG-3'

500 pmol aqueous solution of the target nucleic acid or aqueous solution to which the target nucleic acid was not added (two types), and two types of aqueous solution of nucleic acid probe: the nucleic acid probe of Sequence 3 and the nucleic acid probe of Sequence 4, each 100 pmol, were mixed according to the following combination, added into 100 µl of buffer solution of 25 mM magnesium chloride, 50 mM Tris-HCl (pH 7.2), and were incubated for 3 hours (the concentration of magnesium ion was 25 mM). This reaction liquid was analyzed by acrylamide electrophoresis, and cleaved bands were measured using a densitograph (available from ATTO Corporation).

In the reaction liquid into which only aqueous solution of the nucleic acid probe of Sequence 3 (TASC 1 probe) was added, Kobs-was 1.9 × 10⁻³ min⁻¹. In the case where the nucleic acid probe of Sequence 3 and the target nucleic acid of Sequence 6 were added, Kobs⁺ was 5.3 × 10⁻³ min⁻¹. Parameter as an allosteric effector: Kobs⁺/Kobs- was 3, indicating that self-cleavage of the cleavage portion by the cleavage active region takes place in case of the target nucleic acid alone.

In the reaction liquid into which only aqueous solution of the nucleic acid probe of Sequence 4 (Locked TASC 2 probe: Sequence at 5' end is 3'GAAAAA5') was added, the Kobs⁻ was less than or equal to 1.0 × 10⁻⁵ mm⁻¹, and in the case where the nucleic acid probe of Sequence 4 and the target nucleic acid of Sequence 6 were added, Kobs⁺ was 4.4 × 10⁻³ min⁻¹. Parameter as an allosteric effector: Kobs⁺/Kobs⁻ was 440 or more. When the reaction time was set to 24 hour, the self-cleavage was less than or equal to 3% in the reaction liquid into which only nucleic acid probe of Sequence 4 was added. From this result, it was found that when the nucleic acid probe comprised the stem loop structure, a false positive reaction, in which the cleavage of the cleavage portion by the cleavage active region takes place before hybridization to the target nucleic acid, was improved.

### (Example 3)

The target nucleic acid was detected in the same way as in Example 2 except that, in Example 2, the nucleic acid probe of Sequence 3 and the target nucleic acid of Sequence 6 were not used, but the nucleic acid probe of Sequence 4 (Locked TASC 2 probe), nucleic acid probe of Sequence 5 (Locked TASC 3 probe), a nucleic acid sequence of the following Sequence 8 (different by 2 bases from the complementary strand to the nucleic acid probe of Sequence 5) and a target nucleic acid of the following Sequence 9 (different by 1 base from the complementary strand to the nucleic acid probe of Sequence 5) were used.
(Sequence 8)
5'-GACACTGTCCAGACACCTACGG-3'
(Sequence 9)
5'-GACACTGTCCAGACTCCTACGG-3'

The target nucleic acid of Sequence 8 differed by 2 bases from the sequence complementary to the complementary strand portion of the nucleic acid probe, and the target nucleic acid of Sequence 9 differed by 1 base from the sequence complementary to the complementary strand portion of the nucleic acid probe.

In the reaction liquid into which a solution of the nucleic acid probe of Sequence 4 (Locked TASC 2 probe) and solution of the target nucleic acid of Sequence 6 were added; Kobs (full⁺) was 4.4 × 10⁻³ min⁻¹. In the reaction liquid into which a solution of the nucleic acid probe of Sequence 4 (Locked TASC 2 probe) and solution of the target nucleic acid of Sequence 8 were added, Kobs (1-mis⁺) was 4.0 × 10⁻³ min⁻¹. In the reaction liquid into which a solution of the nucleic acid probe of Sequence 4 (Locked TASC 2 probe) and the target nucleic acid of Sequence 9 were added, Kobs (2-mis⁺) was less than 1.0 × 10⁻⁵ min⁻¹.

In the reaction liquid into which a solution of the nucleic acid probe of Sequence 5 (Locked TASC 3 probe) and solution of the target nucleic acid of Sequence 6 were added, Kobs (full⁺) was 4.3 × 10⁻³ min⁻¹. In the reaction liquid into which a solution of the nucleic acid probe of Sequence 5 (Locked TASC 3 probe) and the target nucleic acid of Sequence 8 were added, Kobs (1-mis⁺) was 1 × 10⁻⁵ min⁻¹. In the reaction liquid into which a solution of the nucleic acid probe of Sequence 5 (Locked TASC 3 probe) and the target nucleic acid of Sequence 9 were added, Kobs (2-mis⁺) was less than or equal to 1.0 × 10⁻⁵ min⁻¹. Parameter as an allosteric effector: Kobs (full)/ Kobs-(1-mis⁺) was 440 or more.

From these results, it was found that the nucleic acid probe of Sequence 5 (Locked TASC 3 probe) did not react if the target nucleic acid was different even by one base and had very high selectivity for the target nucleic acid, and found that as the number of bases in the sequence of the regions hybridizing with the target nucleic acid, 8+8 (2 regions) = 16 (total) was particularly preferable.

### (Example 4)

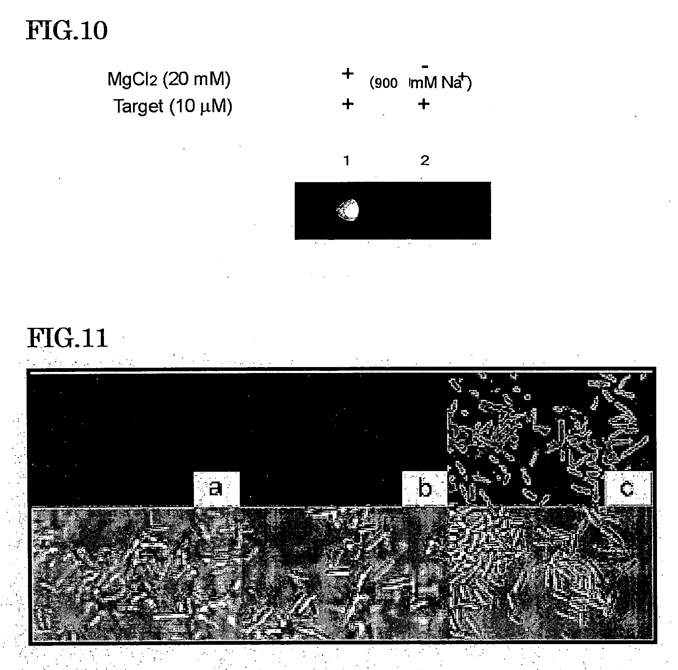

A solution of the nucleic acid probe of Sequence 5 (Locked TASC 3 probe) and solution of the target nucleic acid of Sequence 6 were allowed to react in the same way as in Example 2 except that, in Example 3, a solution of the nucleic acid probe of Sequence 5 (Locked TASC 3 probe) was used, the nucleic acid probe of Sequence 4 (Locked TASC 2 probe) was not used, instead of 100 µl of buffer solution of 25 mM magnesium chloride, 50 mM Tris-HCl (pH 7.2), 100 µl of buffer solution of 900 mM NaCl, 50 mM Tris-HCl (pH 7.2) was used in equivalent amount. Reaction liquid was spotted on the well of a fluorescein 96-well microplate (available from Corning Incorporated), and was observed with a fluorescence-imager (Versadoc 3000 fluorescein imager, available from Bio-Rad Laboratories, Inc.) The results are shown in FIG.10.

From this result, it was found that in the case where magnesium ion was present, cleavage active region of the nucleic acid probe exhibited cleavage activity (See, lane 1 of FIG. 10), but in the case where sodium ion was present, cleavage active region of the nucleic acid probe did not exhibit cleavage activity (See, lane 2 of FIG. 10).

### (Example 5)

E. coli was cultured until the value of OD₆₀₀ of cell suspension had reached 0.5. 167 µl of the cell suspension was centrifuged at 4,000 rpm for 10 minutes. Then, supernatant was removed, precipitated cells were fixed with 4% paraformaldehyde for one hour, washed with PBS, and prepared mixtures containing 1µM nucleic acid probe of Sequence 5 (Locked TASC 3 probe), Tris-HCl (50 mM, pH 7.2, 0.1% SDS) (containing 25 mM MgCl or 0.9 M NaCl, and 10 µM helper oligonucleotide or not containing helper oligonucleotide). These mixtures (three kinds: mixture in which the 25 mM MgCl was present and the aqueous solution of helper oligonucleotide was not added, mixture in which the 0.9 M NaCl was present and the aqueous solution of helper oligonucleotide was added, and mixture in which the 25 mM MgCl was present and the aqueous solution of helper oligonucleotide was added) were incubated at 37°C. 3 µl of the mixtures were centrifuged several times, and were observed using a fluorescence microscope (available from KS Olympus Co., Ltd.).

The base sequences of the helper oligonucleotide are shown in the following Sequences 10 and 11.
(Sequence 10)
3'-GTCGGTGTGACCTTGACTCT-5'
(Sequence 11)
3'-CCCTCCGTCGTCACCCCTTA-5'

These results are shown in FIG. 11. "a" (left picture) of FIG. 11 is a picture of the mixture into which the aqueous solution of the helper oligonucleotide was not added and the MgCl aqueous solution was added. No emission was observed. "b" (middle picture) of FIG. 11 is a picture of the mixture into which the aqueous solution of the helper oligonucleotide and the NaCl aqueous solution was added. Only weak fluorescence was observed. "c" (right picture) of FIG. 11 is a picture of the mixture into which the aqueous solution of the helper oligonucleotide and the MgCl aqueous solution was added. Strong emission was observed.

From these results, it was confirmed that the nucleic acid probe could be introduced into bacteria, and emission was amplified by the addition of the helper oligonucleotide and the MgCl (presence of magnesium ion).

### (Example 6)

Nucleic acid probes of Example 6 were synthesized in the same way as in Example 2 except that, in Example 2, nucleic acid probes having the base sequences of the following Sequences 12 to 15 were designed.
(Sequence 12)
5'-AAAAAGCCGTAGGAGTGCTAGTCTTTTT-3'
(Sequence 13)
5'-CCGTAGGAGTGCTTTTTAGCGGACCGTGTC-3'
(Sequence 14)
5'-AAAAAGCCGTAGGAGTGCTA-3'
(Sequence 15)
5'-GAAGCGGACCGTGTC-3'

The nucleic acid probes of Sequences 12 to 15 are model nucleic acid probes of the nucleic acid probes of Sequences 4 and 5 (having a stem loop structure).

Oligonucleotides having the following sequences were prepared as the target nucleic acids.
(Sequence 16)
5'-GGCATCCTCAGACCTGGCACAG-3'
(Sequence 17)
5'-CATCCTCAGACCTGGCAC-3'

The number of bases in the sequence of the nucleic acid probe of Sequence 13 hybridizing to the target nucleic acid of Sequence 16 was 10 + 10 (2 regions) = 20 (total), and the number of bases in the sequence of the nucleic acid probe of Sequence 13 hybridizing to the target nucleic acid of Sequence 17 was 8 + 8 (2 regions) =16 (total).

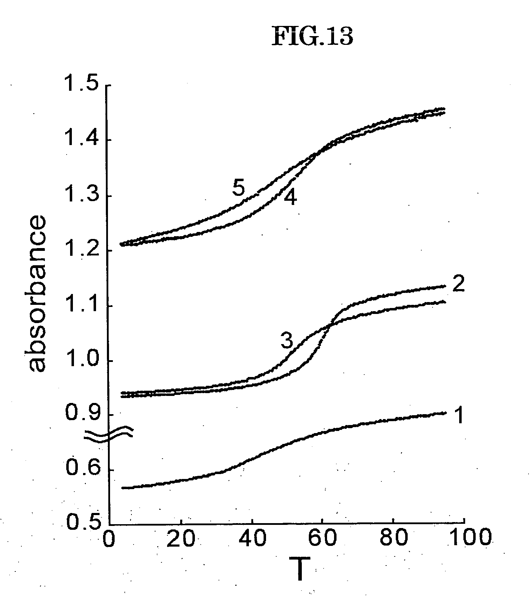

Absorbance at 260 nm of reaction liquids was measured in the same way as in Example 2, except that, in Example 2, the nucleic acid probes of Sequences 12 to 15 were used in place of the nucleic acid probes of Sequences 3 to 5, the target nucleic acids of Sequences 16 and 17 were used in place of the target nucleic acids of Sequences 6 and 7, temperature was changed, and reaction liquids were prepared composed of the combinations of the nucleic acid probe and the target nucleic acid shown below. Relationship between absorbance and temperature is shown in FIG.13.

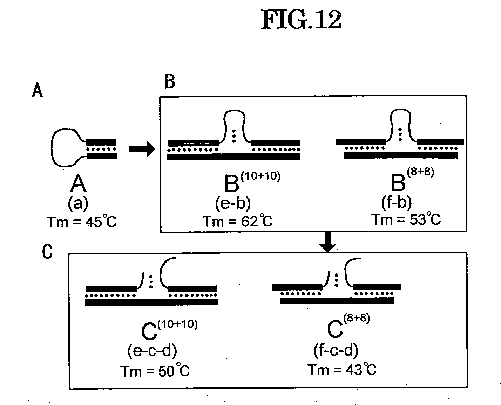

The nucleic acid probe of Sequence 12 had a complete stem loop structure before hybridization to a target nucleic acid (FIG. 12A), and Tm value at the center of the curve of line 1 shown in FIG. 13 was 43°C. From this result, it was found that the stem loop structure in the nucleic acid probes of Sequences 4 and 5 had temperature resistance.

The nucleic acid probe of Sequence 13 and the target nucleic acid of Sequence 16 were allowed to hybridize and the result is shown as line 2 of FIG. 13, and the nucleic acid probe of Sequence 13 and the target nucleic acid of Sequence 17 were allowed to hybridize and the result is shown as line 3 of FIG. 13 (form of FIG. 12B). Tm value at the center of the curve of line 2 was 62°C, and Tm value at the center of the curve of line 3 was 53°C.

The nucleic acid probes of Sequences 14 and 15, and the target nucleic acid of Sequence 16 were allowed to hybridize and the result is shown as line 4 of FIG.13, and the nucleic acid probes of Sequences 14 and 15 and the target nucleic acid of Sequence 17 were allowed to hybridize and the result is shown as line 5 of FIG. 13 (form of FIG. 12C). Tm value at the center of the curve of line 4 was 50°C, and Tm value at the center of the curve of line 5 was 43°C.

From these results, it was found that all of Tm values were 37°C or more and that hybridization occurred without problems at room temperature.

### (Example 7)

A nucleic acid probe of Example 7 was synthesized in the same way as in Example 2, except that, in Example 2, a base sequence of the following Sequence 18 was designed.

In Sequence 18, "rA" as the cleavage portion was located at the 20th position from the 5' end. Further, in Sequence 18, thymine (dT) to which a quenching substance (Q): dimethylaminobenzenesulfonyl (DABSYL) as the quenching portion bound, i.e., dimethylaminobenzenesulfonyl (DABSYL)-dT (available from Glen Research Corporation) was used as the 28th base (T (dT)) from the 5' end, and a fluorescent substance (F): fluorescein as the light-emitting portion was allowed to bind to the 5' end, i.e., fluorescein (available from Glen Research Corporation) was allowed to bind. In this way, the nucleic acid probe of Sequence 18 in Example 7 was prepared.

(Sequence 18)

5′-AAAAAGACTTCTCATCGCTrAGTCTTTTTTTTTGATCCG

AGCCGGACGAAGCCTAGTTGGTC-3′

HeLa cells (uterus cancer cells) were cultured in Dulbecco's medium for one day, and propagation was confirmed with a microscope. 100 microliter was pipetted into each well of 96 well plate after confirmation of each having a concentration of 1 × 10⁵ cells /ml. After washing with a 100 microliter of PBS buffer, the nucleic acid probe of Example 7 (1 microliter) targeting c-fos gene was added together with lipofectin (available from Invitrogen Corporation), and cultured at 37°C for 1 hour. Fluorescence spectrum was detected using a fluorescence microscope (FV500, available from KS Olympus Co., Ltd.).

Emission was not detected from normal cells used as control, however, noticeable fluorescence amplification was observed from the Hela cells. From this result, it was found that the nucleic acid probe could be also used in live cells of higher organisms, useful information on the diagnosis of the cancer cells and normal cells could be obtained.

### (Example 8)

HeLa cells (uterus cancer cells) were cultured in Dulbecco's medium for one day, and propagation was confirmed with a microscope. 100 microliter was pipetted into each well of 96 well plate after confirmation of each having a concentration of 1 × 10⁵ cells /ml. Growth factor EGF and tyrosine kinase inhibitor were added to a culture medium, and cultured at 37°C for 6 hour. After washing with a 100 microliter of PBS buffer, a nucleic acid probe of Example 8 (1 microliter) targeting EGFR signal marker c-fos gene was added together with lipofectin (available from Invitrogen Corporation), and cultured at 37°C for 1 hour. Fluorescence spectrum was detected using a fluorescence microscope (FV500, available from KS Olympus Co., Ltd.). Emission was not detected from cells, to which tyrosine kinase inhibitor was not added, used as control, however, noticeable fluorescence amplification was observed from cells to which the tyrosine kinase inhibitor was added.

From this result, it was found that in the Hela cells after introduction of tyrosine kinase inhibitor, expression of original cancer gene was suppressed. This indicates that by using the nucleic acid probe of the invention, useful information on screening of medicines can be obtained.

### (Example 9)

The nucleic acid probe of Sequence 5 and target nucleic acid of Sequence 6 were allowed to react in the same way as in Example 2, except that, in Example 2, the nucleic acid probe of Sequence 5 (Locked TASC 3 probe) was used, reactions were carried out in 1mM MgCl aqueous solution, 5mM MgCl aqueous solution, 10mM MgCl aqueous solution, 15mM MgCl aqueous solution, 20mM MgCl aqueous solution, or 30mM MgCl aqueous solution (each concentration was final concentration). Reaction liquids were spotted on a fluorescein 96-well microplate (available from Corning Incorporated), and were observed with a Versadoc 3000 fluorescein imager (available from Bio-Rad Laboratories, Inc.).

From this result, it was found that preferable concentration of MgCl to be added was 20 mM to 30 mM.

### Industrial Applicability

The invention can provide a nucleic acid probe which has a very high selectivity for a target nucleic acid, which can detect a trace amount of target nucleic acid highly sensitively, highly accurately, and highly quickly by amplifying emission generated from the nucleic acid probe hybridized to the target nucleic acid, and which is suitable for a gene diagnosis, test for the presence of food-poisoning bacteria, diagnosis of tooth decay or periodontal disease, blood test, etc.

The nucleic acid probe of the invention is particularly suitable for, e.g. detection of a trace amount of target nucleic acid (gene) of, for example, viruses, bacteria, animal cells, plant cells, is particularly suitable for a test for prevention of viral infection of blood for blood transfusion and for analysis of the condition of a patient with nervous disease, and can improve the life-span and QOL of humans. The nucleic acid probe is advantageous, for example, in that there is no need for enzyme or reagent, heating, etc. is not required and the probe functions even at living body temperature, there is no need for amplification of the signal as much as causing cell death, and the target nucleic acid can be detected without destroying cells.

The invention also can provide a nucleic acid chip which can detect or analyze a trace amount of target nucleic acid highly sensitively, highly accurately, and highly quickly by immobilizing the nucleic acid probe, and which is suitable for a gene diagnosis, test for the presence of food-poisoning bacteria, diagnosis of tooth decay or periodontal disease, blood test, etc. Since the nucleic acid chip uses the nucleic acid probe of the invention, the effect of the nucleic acid probe can be achieved as it is.

The invention also can provide a method for detecting a target nucleic acid and an apparatus for detecting a target nucleic acid which can detect or analyze a trace amount of target nucleic acid highly sensitively, highly accurately, and highly quickly by using the nucleic acid probe. The method for detecting a target nucleic acid and the apparatus for detecting a target nucleic acid of the invention can improve QOL of a patient in a remote area, and patients living in the countryside and in a country where the distance from home to the hospital is long by cutting out the need to go to the hospital. Since the method for detecting a target nucleic acid and the apparatus for detecting a target nucleic acid use the nucleic acid probe of the invention, the effect of the nucleic acid probe can be achieved as it is.

The invention also can provide a method for screening a drug which can analyze an effect of administration of a drug by using the nucleic acid probe, and which can screen a desired drug efficiently. The method for screening a drug of the invention is a revolutionary technique that can revolutionize in drug industry, which technique makes side effect and mechanism of action clear and makes tailor-made medicine realize, from genomic information which was not available until now. Since the method for screening a drug uses the nucleic acid probe of the invention, the effect of the nucleic acid probe can be achieved as it is.

The invention also can provide a gene diagnosis method which can diagnose whether or not one is a patient of a specific disease highly efficiently and highly accurately by using the nucleic acid probe. The gene diagnosis method of the invention can improve QOL of a patient in a remote area, and patients living in the countryside and in a country where the distance from home to the hospital is long by cutting out the need to go to the hospital. Since the gene diagnosis method uses the nucleic acid probe of the invention, the effect of the nucleic acid probe can be achieved as it is.

## Claims

1. A nucleic acid probe for detecting a target nucleic acid,
wherein the nucleic acid probe is designed so that the nucleic acid probe undergoes a conformational change after hybridization to the target nucleic acid, the nucleic acid probe, which underwent the conformational change, has a decreased binding strength of hybridization and dissociates from the target nucleic acid.

2. The nucleic acid probe according to claim 1, wherein the conformational change comprises formation of a self-cleaving nucleic acid enzyme.

3. The nucleic acid probe according to one of claims 1 and 2, wherein the nucleic acid probe comprises a labeling substance.

4. A nucleic acid probe for detecting a target nucleic acid, comprising:
a complementary region having a sequence complementary to at least a portion of the base sequence of the target nucleic acid; and
a nucleic acid enzyme forming region capable of forming a self-cleaving nucleic acid enzyme.

5. The nucleic acid probe according to claim 4, wherein the self-cleaving nucleic acid enzyme has cleavage activity, by which the nucleic acid probe is cleaved specifically.

6. The nucleic acid probe according to claims 4 and 5, further comprising at least one intramolecular hybridization region which comprises a sequence capable of hybridizing within the molecule of the nucleic acid probe.

7. The nucleic acid probe according to any one of claims 4 to 6, further comprising a labeling substance which makes it easier to recognize the presence of the target nucleic acid.

8. The nucleic acid probe according to claim 7, wherein the labeling substance comprises at least a pair of a fluorescent substance and a quenching substance.

9. The nucleic acid probe according to any one of claims 1 to 8, which is immobilized on a support.

10. A nucleic acid probe, comprising a conformation variable portion,
wherein the nucleic acid probe is capable of hybridizing to a target nucleic acid and the conformation of the conformation variable portion is capable of changing upon hybridization to the target nucleic acid, and
wherein the nucleic acid probe is capable of dissociating from the target nucleic acid when the conformation of the conformation variable portion has changed.

11. A nucleic acid probe, comprising a cleavage portion,
wherein the nucleic acid probe is capable of hybridizing to a target nucleic acid and the cleavage portion is cleaved upon hybridization to the target nucleic acid, and
wherein the nucleic acid probe is capable of forming a cleavage active region having cleavage activity toward the cleavage portion upon hybridization to the target nucleic acid.

12. The nucleic acid probe according to one of claims 10 and 11, further comprising a signal-generating portion from which a signal is generated.

13. A nucleic acid probe, comprising:
a cleavage portion; and
a signal-generating portion from which a signal is generated,
wherein the nucleic acid probe is capable of hybridizing to a target nucleic acid and the cleavage portion is cleaved upon hybridization to the target nucleic acid and generates a first probe fragment and a second probe fragment which are capable of dissociating from the target nucleic acid.

14. The nucleic acid probe according to one of claims 12 and 13, wherein a signal is generated from the signal-generating portion when the first probe fragment and the second probe fragment have dissociated from the target nucleic acid.

15. The nucleic acid probe according to any one of claims 12 to 14, wherein the signal-generating portion comprises a light-emitting portion generating emission and a quenching portion which quenches the emission of the light-emitting portion when the quenching portion is located adjacent to the light-emitting portion.

16. The nucleic acid probe according to claim 15, wherein the light-emitting portion is present in the first probe fragment, and the quenching portion is present on the second probe fragment.

17. The nucleic acid probe according to any one of claims 1 to 16, wherein the nucleic acid probe has formed a locked structure as a result of intramolecular hybridization of the portions of the nucleic acid probe to each other until the nucleic acid probe hybridizes to the target nucleic acid.

18. A nucleic acid probe, comprising a cleavage portion,
wherein the nucleic acid probe is capable of hybridizing to a target nucleic acid and the cleavage portion is cleaved upon hybridization to the target nucleic acid, and
wherein the nucleic acid probe has formed a locked structure as a result of intramolecular hybridization of portions of the nucleic acid probe to each other until the nucleic acid probe hybridizes to the target nucleic acid.

19. The nucleic acid probe according to one of claims 17 and 18, wherein the locked structure is at least one selected from the group consisting of a stem loop structure, a hairpin structure, and an internal loop structure.

20. The nucleic acid probe according to any one of claims 17 to 19,
wherein the locked structure comprises at least one intramolecular hybridization region in which intramolecular hybridization has occurred, and
wherein, in the intramolecular hybridization region, a base sequence of one complementary strand is 3 nt to 8 nt in length.

21. The nucleic acid probe according to any one of claims 1 to 20, comprising at least one intramolecular hybridization region, portions of which are capable of hybridizing to each other intramolecularly when the nucleic acid probe has hybridized to the target nucleic acid.

22. The nucleic acid probe according to claim 21, comprising two or more intramolecular hybridization regions,
wherein the total length of base sequences of Y and Z is 2 nt to 12 nt, where the Y and the Z are the intramolecular hybridization regions, and are located adjacent to each other via a cleavage portion.

23. The nucleic acid probe according to claim 22, wherein the base sequence of the Y is 1 to 8 in length, and the base sequence of the Z is 1 to 8 in length.

24. The nucleic acid probe according to any one of claims 1 to 20, comprising at least two base sequences complementary to the target nucleic acid,
wherein the at least two base sequences are 6 bases to 10 bases in length.

25. The nucleic acid probe according to any one of claims 11, 13 and 18, wherein the cleavage portion comprises a nucleotide, and sugar of the nucleotide is ribose.

26. The nucleic acid probe according to any one of claims 11, 13 and 18, wherein the nucleic acid probe is capable of forming a cleavage active region exhibiting cleavage activity toward the cleavage portion upon hybridization to the target nucleic acid.

27. The nucleic acid probe according to claim 26, wherein the cleavage active region has at least one of DNAzyme activity, RNAzyme activity, and ribozyme activity.

28. The nucleic acid probe according to claim 27, wherein the cleavage active region exhibits cleavage activity toward the cleavage portion by interacting with a metal ion.

29. The nucleic acid probe according to claim 28, wherein the metal ion is a magnesium ion.

30. The nucleic acid probe according to any one of claims 15 to 29,
wherein the light-emitting portion comprises a light-emitting substance and the quenching portion comprises a quenching substance, and
wherein a combination of the light-emitting substance and the eliminating substance is selected from a combination of fluorescein isothyocianate (FITC) and tetramethylrhodamine isothiocyanate (TRITC), and a combination of dimethylamino-azobenzene-sulfonyl (DABSYL) and fluorescein.

31. A nucleic acid chip comprising:
a support; and
the nucleic acid probe of any one of claims 1 to 30 immobilized on the support.

32. A method for detecting a target nucleic acid using a nucleic acid probe, wherein the nucleic acid probe is designed such that it undergoes a conformational change after hybridization to the target nucleic acid, the nucleic acid probe, which underwent the conformational change, has a decreased binding strength of hybridization and dissociates from the target nucleic acid.

33. The method for detecting a target nucleic acid according to claim 32, wherein the conformational change comprises formation of a self-cleaving nucleic acid enzyme.

34. The method for detecting a target nucleic acid according to one of claims 32 and 33, wherein the nucleic acid probe comprises a labeling substance, and the labeling substance emits different signals before and after the nucleic acid probe dissociates from the target nucleic acid.

35. A method for detecting a target nucleic acid using a nucleic acid probe which comprises: a complementary region having a sequence complementary to at least a portion of the base sequence of the target nucleic acid; and a nucleic acid enzyme forming region capable of forming a self-cleaving nucleic acid enzyme,
wherein the method for detecting a target nucleic acid comprises:
a hybridization step in which the nucleic acid probe is allowed to bind to the target nucleic acid complementarily;
a dissociation step in which the self-cleaving nucleic acid enzyme is formed after the formation of hybridization, the nucleic acid probe undergoes a conformational change, as a result, the binding strength of hybridization decreases, and the nucleic acid probe dissociates from the target nucleic acid;
and a detection step in which at least a part of the nucleic acid probe dissociated in the dissociation step is detected.

36. The method for detecting a target nucleic acid according to claim 35, wherein the nucleic acid probe is cleaved by the self-cleaving nucleic acid enzyme.

37. The method for detecting a target nucleic acid according to one of claims 35 and 36, wherein the nucleic acid probe is immobilized on a support.

38. A method for detecting a target nucleic acid, comprising:
a hybridization step in which the nucleic acid probe of any one of claims 1 to 30 is allowed to hybridize to the target nucleic acid; and
a target nucleic acid detection step in which the presence of the target nucleic acid is detected by detecting a signal generated from the nucleic acid probe upon hybridization to the target nucleic acid.

39. The method for detecting a target nucleic acid according to claim 38, wherein, a first probe fragment and a second probe fragment are generated by the cleavage of the nucleic acid probe upon hybridization to the target nucleic acid, and in the target nucleic acid detection step, the presence of the target nucleic acid is detected by detecting the emission of a light-emitting portion which is present in the first probe fragment.

40. The method for detecting a target nucleic acid according to one of claims 38 and 39, wherein the target nucleic acid is one of a nucleic acid present in a cell or a nucleic acid of a virus.

41. The method for detecting a target nucleic acid according to claims 38 and 40, the hybridization to the target nucleic acid is carried out under the presence of a helper oligonucleotide.

42. A method for screening a drug, comprising:
a hybridization step in which the nucleic acid probe of any one of claims 1 to 23 is allowed to hybridize to a target nucleic acid expressed due to administration of the drug; and
a target nucleic acid detection step in which the presence or absence of the target nucleic acid is detected by detecting the presence or absence of a signal generated from the nucleic acid probe upon hybridization to the target nucleic acid,
wherein the drug is screened by the presence or absence of the target nucleic acid.

43. The method for screening a drug according to claim 42, wherein, a first probe fragment and a second probe fragment are generated by the cleavage of the nucleic acid probe upon hybridization to the target nucleic acid, and in the target nucleic acid detection step, the presence of the target nucleic acid is detected by detecting the emission of a light-emitting portion which is present in the first probe fragment.

44. An apparatus for detecting a target nucleic acid, which is a device for analyzing a nucleic acid in which a nucleic acid probe for detecting a target nucleic acid in a sample is immobilized on a support,
wherein the nucleic acid probe is designed so that the nucleic acid probe undergoes a conformational change after hybridization to the target nucleic acid, the nucleic acid probe, which underwent the conformational change, has a decreased binding strength of hybridization and dissociates from the target nucleic acid.

45. The apparatus for detecting a target nucleic acid according to claim 44, wherein the conformational change comprises formation of a self-cleaving nucleic acid enzyme.

46. The apparatus for detecting a target nucleic acid according to one of claims 44 and 45, wherein the nucleic acid probe comprises a labeling substance which makes it easier to recognize the target nucleic acid.

47. A apparatus for detecting a target nucleic acid, which is a device for analyzing a nucleic acid for detecting a target nucleic acid in a sample,
wherein a nucleic acid probe is immobilized on a support, and
wherein the nucleic acid probe comprises:
a complementary region having a sequence complementary to at least a portion of the base sequence of the target nucleic acid; and
a nucleic acid enzyme forming region capable of forming a self-cleaving nucleic acid enzyme.

48. The apparatus for detecting a target nucleic acid according to claim 47,
wherein the self-cleaving nucleic acid enzyme has cleavage activity, by which the nucleic acid probe is cleaved specifically.

49. The apparatus for detecting a target nucleic acid according to any one of claims 44 to 48, wherein the nucleic acid probe comprises at least one intramolecular hybridization region which comprises a sequence capable of hybridizing within the molecule of the nucleic acid probe.

50. The apparatus for detecting a target nucleic acid according to any one of claims 44 to 49, wherein the nucleic acid probe comprises a labeling substance which makes it easier to recognize the target nucleic acid.

51. A apparatus for detecting a target nucleic acid comprising:
a unit configured to allow the nucleic acid probe of any one of claims 1 to 30 to hybridize to the target nucleic acid; and
a target nucleic acid detector configured to detect the presence of the target nucleic acid by detecting a signal generated from the nucleic acid probe upon hybridization to the target nucleic acid.

52. The apparatus for detecting a target nucleic acid according to claim 51, wherein, a first probe fragment and a second probe fragment are generated by the cleavage of the nucleic acid probe upon hybridization to the target nucleic acid, and the target nucleic acid detector detects the emission of a light-emitting portion which is present in the first probe fragment to thereby detect the presence of the target nucleic acid.

53. The apparatus for detecting a target nucleic acid according to one of claims 51 and 52, further comprising:
a database part which stores database of genomic information; and
a data analysis part which compares and analyzes data of the result of detection by the target nucleic acid detector and genomic information data stored in the database part.

54. The apparatus for detecting a target nucleic acid according to any one of claims 51 to 53, further comprising:
a communication part capable of communicating with internet, and capable of accessing to database of genomic information on the internet; and
a data analysis part which compares and analyzes data of the result of detection by the target nucleic acid detector and genomic information data of the database of genomic information on the internet.

55. The apparatus for detecting a target nucleic acid according to any one of claims 51 to 54, wherein a database of genomic information comprises genomic information of a healthy individual and genomic information of a patient, related to a particular disease.

56. The apparatus for detecting a target nucleic acid according to claim 55, wherein the target nucleic acid detector determines the amount of the target nucleic acid quantitatively.

57. A gene diagnosis method using the apparatus for detecting a target gene of claim 56,
wherein the gene diagnosis method comprises:
a target nucleic acid expression level quantitative determination step in which the expression level of a target nucleic acid related to a specific disease in a subject is determined quantitatively; and
a diagnosis step in which the expression level of the target nucleic acid related to the specific disease in the subject, and the expression levels, in a healthy individual and in a patient, of the target nucleic acid related to the specific disease which are contained in a database of genomic information, are compared by a data analysis part to diagnose whether or not the subject is a patient of the specific disease.

58. The gene diagnosis method according to claim 57, wherein, in the diagnosis step, if (Xi-S/M-S) >_ 0.9 is satisfied, the subject is diagnosed as the patient of the specific disease, where M: expression level of the target gene related to the specific disease, of the patient, S: expression level of the healthy individual, and Xi: expression level of the subject.
